# EUROPEAN PATENT APPLICATION

(11) **EP 4 159 843 A1**
(43) Date of publication of application: **05.04.2023**
(21) Application number: 21811798.4
(22) Date of filing: 28.05.2021
(51) Int. Cl.: C12N 5/0783, A61K 35/17, A61P 35/00

(54) **SEED CELL MEDIUM OF TUMOR-INFILTRATING LYMPHOCYTE AND APPLICATION THEREOF**

(30) Priority: 29.05.2020 CN 202010476302
(71) Applicant: Shanghai Juncell Therapeutics Co., Ltd., Jiading District, Shanghai 201800 (CN)
(72) Inventor: JIN, Huajun, Shanghai 201800 (CN); HE, Zhou, Shanghai 201800 (CN); MA, Xingming, Shanghai 201800 (CN); LI, Tiantian, Shanghai 201800 (CN); HUANG, Chen, Shanghai 201800 (CN)
(74) Representative: Böhm, Brigitte
(86) International application number: PCT/CN2021/096585
(87) International publication number: WO 2021/239083

(57) **Abstract**

The present application relates to a seed cell medium of a tumor-infiltrating lymphocyte and an application thereof. The medium comprises the following components: a cell culture ingredient, a cell factor, and an immune checkpoint antibody or an antigen-binding fragment thereof, wherein the cell factor comprises IL-2, an immune checkpoint comprises: PD-1, LAG-3, TIGIT, and/or CTLA-4, and the cell culture component is a serum medium or a serum-free medium. The seed cell medium of the present application can accelerate culture of a seed cell of the tumor-infiltrating lymphocyte, and/or shorten the amplification time required by the tumor-infiltrating lymphocyte.

## Description

### FIELD OF THE INVENTION

The present application relates to the field of biomedicine, and in particular, relates to a seed cell medium for tumor-infiltrating lymphocytes and an application thereof.

### BACKGROUND OF THE INVENTION

In 1998, the Surgery Branch of the National Cancer Institute (NCI) in the United States reported the first case of treating a patient with tumor-infiltrating lymphocytes (TIL-). Extensive studies on TIL- have shown that cells with anti-tumor activity can be isolated from tumor tissues of patients with melanoma. In recent years, it has been found that TIL- has a very significant therapeutic effect for tumors, in particular solid tumors, that are large-volume and incurable with respect to adoptive transfer therapy (ACT).

When TIL- is used in tumor immunotherapy, a large amount of cells are required, which presents a great challenge in terms of technology and process control for the expansion of TIL- cells. The common practice in the past included a pre-rapid expansion procedure (pre-REP) based on IL-2, followed by a "rapid expansion procedure" (REP). This procedure requires a large amount of excessive irradiated allogeneic PBMCs (also called mononuclear cells, MNC) from multiple donors as feeder cells, as well as an anti-CD3 antibody (OKT3) and high-dose IL-2. The irradiated allogeneic PBMCs from multiple donor sources have a potential risk of contamination, and the IL-2 at a high concentration may have a risk of cell exhaustion in the subsequent infusion.

Therefore, there is an urgent need to provide a medium that facilitates the quick acquisition of seed cells of tumor-infiltrating lymphocytes.

### SUMMARY OF THE INVENTION

The present application provides a seed cell medium for tumor-infiltrating lymphocytes and an application thereof. The seed cell medium of the present application and the preparation method therefor have at least one beneficial effect selected from the group consisting of: 1) significantly reducing the dose of IL-2; 2) reducing or even eliminating the need to use IL-2 in subsequent infusion; 3) reducing the risk of cell exhaustion caused by the high-concentration IL-2 in the subsequent infusion; 4) eliminating the need of using exogenous allogeneic PBMCs or other cells as feeder cells in the subsequent REP, and reducing exogenous contamination; 5) promoting the subsequent REP to reach the yield of 10¹⁰-10¹¹ cells; and 6) significantly increasing the efficiency of obtaining seed cells and even that of expanding the tumor-infiltrating lymphocytes as well as shortening the culture time.

In one aspect, the present application provides a seed cell medium for tumor-infiltrating lymphocytes, comprising the following components: a cell culture ingredient, a cytokine, and an immune checkpoint antibody or an antigen-binding fragment thereof, wherein said cytokine comprises IL-2, said immune checkpoint comprises: PD-1, LAG-3, TIGIT and/or CTLA-4, and said cell culture ingredient is a serum-containing medium or a serum-free medium.

In some embodiments, said IL-2 has a concentration of about 3000 IU/mL or less.

In some embodiments, said IL-2 has a concentration of about 2000 IU/mL to about 3000 IU/mL.

In some embodiments, said cytokine comprises IL-7.

In some embodiments, said IL-7 has a concentration of about 200 U/mL to about 1000 U/mL.

In some embodiments, said cytokine comprises IL-15.

In some embodiments, said IL-15 has a concentration of about 200 U/mL to about 500 U/mL.

In some embodiments, said cytokine comprises a tumor necrosis factor (TNF).

In some embodiments, said cytokine comprises TNFα.

In some embodiments, said TNFα has a concentration of about 10 pg/mL to about 100 pg/mL.

In some embodiments, said cytokine comprises a colony stimulating factor.

In some embodiments, said cytokine comprises GM-CSF, G-CSF and/or M-CSF.

In some embodiments, said GM-CSF has a concentration of about 200 U/mL to about 5000 U/mL.

In some embodiments, said G-CSF has a concentration of about 300 U/mL to about 1000 U/mL.

In some embodiments, said M-CSF has a concentration of about 300 U/mL to about 800 U/mL.

In some embodiments, said cytokine comprises an interferon.

In some embodiments, said cytokine comprises IFN-γ, IFN-α and/or IFN-β.

In some embodiments, said IFN-γ has a concentration of about 10 ng/mL to about 1000 ng/mL.

In some embodiments, said IFN-γ has a concentration of about 300 U/mL to about 1000 U/mL.

In some embodiments, said IFN-α has a concentration of about 500 U/mL to about 1000 U/mL.

In some embodiments, said IFN-β has a concentration of about 200 U/mL to about 500 U/mL.

In some embodiments, said cytokine further comprises: IL-4, IL-1α, IL-1β, IL-6, IL-9, IL-18, IL-12, IL-21 and/or IL-10.

In some embodiments, said IL-4 has a concentration of about 200 U/mL to about 500 U/mL.

In some embodiments, said IL-1α has a concentration of about 200 U/mL to about 500 U/mL.

In some embodiments, said IL-1β has a concentration of about 200 U/mL to about 500 U/mL.

In some embodiments, said IL-6 has a concentration of about 200 U/mL to about 500 U/mL.

In some embodiments, said IL-9 has a concentration of about 200 U/mL to about 500 U/mL.

In some embodiments, said IL-18 has a concentration of about 200 U/mL to about 500 U/mL.

In some embodiments, said IL-12 has a concentration of about 200 U/mL to about 1000 U/mL.

In some embodiments, said IL-21 has a concentration of about 200 U/mL to about 500 U/mL.

In some embodiments, said IL-10 has a concentration of about 200 U/mL to about 500 U/mL.

In some embodiments, said immune checkpoint antibody or the antigen-binding fragment thereof is a human PD-1 antibody or an antigen-binding fragment thereof.

In some embodiments, said immune checkpoint antibody or the antigen-binding fragment thereof is a PD-1 antibody or an antigen-binding fragment thereof, and said PD-1 antibody or the antigen-binding fragment thereof has a concentration of about 1 µg/mL to about 100 µg/mL.

In some embodiments, said immune checkpoint antibody or the antigen-binding fragment thereof is a human LAG-3 antibody or an antigen-binding fragment thereof.

In some embodiments, said immune checkpoint antibody or the antigen-binding fragment thereof is a LAG-3 antibody or an antigen-binding fragment thereof, and said LAG-3 antibody or the antigen-binding fragment thereof has a concentration of about 3 µg/mL to about 10 µg/mL.

In some embodiments, said TIGIT antibody or the antigen-binding fragment thereof is a human TIGIT antibody or an antigen-binding fragment thereof.

In some embodiments, said immune checkpoint antibody or the antigen-binding fragment thereof is a TIGIT antibody or an antigen-binding fragment thereof, and said TIGIT antibody or the antigen-binding fragment thereof has a concentration of about 1 µg/mL to about 25 µg/mL.

In some embodiments, said immune checkpoint antibody or the antigen-binding fragment thereof is a human CTLA-4 antibody or an antigen-binding fragment thereof.

In some embodiments, said immune checkpoint antibody or the antigen-binding fragment thereof is a CTLA-4 antibody or an antigen-binding fragment thereof, and said CTLA-4 antibody or the antigen-binding fragment thereof has a concentration of about 1 µg/mL to about 100 µg/mL.

In some embodiments, said seed cell medium further comprises a costimulatory receptor antibody or an antigen-binding fragment thereof, wherein said costimulatory receptor antibody or the antigen-binding fragment thereof comprises: a CD40 antibody or an antigen-binding fragment, an OX-40 antibody or an antigen-binding fragment thereof, a CD137 antibody or an antigen-binding fragment thereof, and/or a CD28 antibody or an antigen-binding fragment thereof.

In some embodiments, said immune checkpoint antibody or the antigen-binding fragment thereof comprises a CD137 antibody or an antigen-binding fragment thereof.

In some embodiments, said CD137 antibody or the antigen-binding fragment thereof is a human CD137 antibody or an antigen-binding fragment thereof.

In some embodiments, said CD137 antibody or the antigen-binding fragment thereof has a concentration of about 1 µg/mL to about 100 µg/mL.

In some embodiments, said immune checkpoint antibody or the antigen-binding fragment thereof comprises a CD28 antibody or an antigen-binding fragment thereof.

In some embodiments, said CD28 antibody or the antigen-binding fragment thereof is a human CD28 antibody or an antigen-binding fragment thereof.

In some embodiments, said CD28 antibody or the antigen-binding fragment thereof has a concentration of about 1 µg/mL to about 10 µg/mL.

In some embodiments, said CD40 antibody or the antigen-binding fragment thereof is a human CD40 antibody or an antigen-binding fragment thereof.

In some embodiments, said CD40 antibody or the antigen-binding fragment thereof has a concentration of about 5 µg/mL to about 10 µg/mL.

In some embodiments, said OX-40 antibody or the antigen-binding fragment thereof is a human OX-40 antibody or an antigen-binding fragment thereof.

In some embodiments, said OX-40 antibody or the antigen-binding fragment thereof has a concentration of about 3 µg/mL to about 10 µg/mL.

In some embodiments, said serum medium comprises a serum.

In some embodiments, said serum comprises a human AB serum.

In some embodiments, said serum has a concentration of about 1% to about 10% (v/v).

In some embodiments, said serum medium comprises an AIM-V medium.

In some embodiments, said serum-free medium comprises an X-VIVO medium.

In some embodiments, said cell culture ingredient comprises antibiotics.

In some embodiments, said cell culture ingredient comprises a penicillin-streptomycin (PS) mixed solution.

In some embodiments, said penicillin-streptomycin (PS) mixed solution has a concentration of about 1 U/mL to about 200 U/mL.

In some embodiments, said seed cell medium further comprises an M2 macrophage inhibitor, said M2 macrophage inhibitor comprising RRx001 and/or CNI-1493.

In some embodiments, said M2 macrophage inhibitor has a concentration of about 0.1 µM to about 100 µM.

In some embodiments, said seed cell medium further comprises a regulatory T cell (Treg) inhibitor, said regulatory T cell inhibitor comprising CAL-101, dasatinib, imatinib and/or panobinostat.

In some embodiments, said regulatory T cell inhibitor has a concentration of about 0.1 µM to about 100 µM.

In some embodiments, said seed cell medium further comprises a bone myeloid-derived suppressor cell (MDSC) inhibitor, said bone myeloid-derived suppressor cell inhibitor comprising AG490, decitabine, sunitinib and/or BBI608.

In some embodiments, said bone myeloid-derived suppressor cell inhibitor has a concentration of about 0.1 µg/mL to about 100 µg/mL.

In some embodiments, said seed cell medium further comprises a T cell activator, said T cell activator comprising LYC-55716, GNE-1858 and/or methylene blue.

In some embodiments, said T cell activator has a concentration of about 1 µM to about 10 µM.

In some embodiments, said seed cell medium further comprises a T cell differentiation inhibitor, said T cell differentiation inhibitor comprising TWS119.

In some embodiments, said T cell differentiation inhibitor has a concentration of about 1 µM to about 10 µM.

In another aspect, the present application provides a seed cell of a tumor-infiltrating lymphocyte obtained by culture using said seed cell medium of the present application, or a cell population thereof.

In another aspect, the present application provides a pharmaceutical composition, comprising said seed cell of the tumor-infiltrating lymphocyte or the cell population thereof according to the present application, and a pharmaceutically acceptable carrier.

In another aspect, the present application provides use of said seed cell of the tumor-infiltrating lymphocyte or the cell population thereof according to the present application, and said pharmaceutical composition according to the present application in the preparation of a medicament for treating a cancer.

In some embodiments, said cancer is selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated pelvic adenocarcinoma, and bile duct cancer.

In another aspect, the present application provides use of said seed cell medium in the expansion of tumor-infiltrating lymphocytes.

In another aspect, the present application provides a method for culturing a seed cell for tumor-infiltrating lymphocytes, comprising the steps of: culturing isolated tumor-infiltrating lymphocytes in the seed cell medium of the present application.

In another aspect, the present application provides a method for expanding tumor-infiltrating lymphocytes, comprises the steps of: culturing isolated tumor-infiltrating lymphocytes in said seed cell medium of the present application to obtain seed cells of said tumor-infiltrating lymphocytes.

In some embodiments, said isolated tumor-infiltrating lymphocytes are derived from a sample selected from the group consisting of: a peritoneal effusion from a subject in need, a primary tumor sample from surgical resection, a metastatic sample from synchronous and metachronous surgical resection, a needle biopsy sample, and a body fluid.

In some embodiments, said body fluid comprises blood, a tissue fluid, a lymph fluid and/or a body cavity effusion.

In some embodiments, said isolated tumor-infiltrating lymphocytes are derived from a tumor selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated adenocarcinoma, and bile duct cancer.

In some embodiments, said isolated tumor-infiltrating lymphocytes are derived from tissue fragments obtained by cutting a tumor tissue resection, and said tissue fragments obtained by cutting a tumor tissue resection has a diameter of about 1 mm to about 10 mm.

In some embodiments, a time for said culturing is about 3-20 days.

In some embodiments, a temperature for said culturing is about 30-42°C.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:
FIGs. 1A-1B to FIGs. 9A-9B show the flow cytometry results of TIL seed cells obtained by culturing from solid tumor tissues using the seed cell medium of the present application;
FIGs. 10A-10B to FIGs. 12A-12B show the flow cytometry results of TIL seed cells obtained by culturing from the body fluid of tumor patients using the seed cell medium of the present application;
FIGs. 13A-13B to FIGs. 15A-15B show the flow cytometry results of TIL seed cells obtained by culturing from the body fluid of tumor patients using the seed cell medium in the prior art;
FIGs. 16A-16B and FIGs. 17-20 show the detection results of high-throughput sequencing analysis of a TIL infusion product prepared using the seed cell medium of the present application and patients' peripheral blood at different time points with respect to TCR repertoire, after the clinical infusion of the TIL infusion product; and
Figure 21 shows the proliferation results of the peripheral blood leukocytes, neutrophils and lymphocytes of patients after clinical infusion using the TIL infusion product prepared using the seed cell medium of the present application.

### DETAILED DESCRIPTION OF THE PREFERRED EMBODIMENTS

The embodiments of the invention of the present application will be illustrated by specific examples below. Those familiar with this technology can easily understand other advantages and effects of the invention of the present application from the content disclosed in the specification.

### TERMS & DEFINITIONS

In the present application, the term "interferon" generally refers to a family of proteins which inhibit virus replication and cell proliferation, regulate immune responses, and have high species specificity. Typical suitable interferons comprise but are not limited to: a recombinant interferon α-2b, a recombinant interferon α-2a, a recombinant interferon α-2C, an interferon α-n1, a consensus α interferon, or an interferon α-n3.

In the present application, the term "colony stimulating factor" generally refers to a type of secretory glycoproteins, which bind to receptor proteins on the surface of hematopoietic stem cells to activate intracellular signaling pathways, which can lead to cell proliferation and cell differentiation into a specific type of blood cells (usually white blood cells, and for red blood cell formation, see erythropoietin). They can be artificially synthesized and exogenously applied.

In the present application, the term "antigen-binding fragment" generally refers to a portion of an antibody molecule that contains amino acids responsible for the specific binding between an antibody and an antigen. The portion of the antigen that is specifically recognized and bound by the antibody is called the "epitope" as described above. As described above, an antigen-binding domain may typically comprise an antibody light-chain variable region (VL) and an antibody heavy-chain variable region (VH); however, it is unnecessary to comprise both. A Fd fragment has, for example, two VH regions and generally retains some of the antigen binding functions of the complete antigen-binding domain. Examples of antigen-binding fragments of antibodies comprise: (1) Fab fragments, which are monovalent fragments having VL, VH, constant light chains (CL) and CH1 domains; (2) F(ab')2 fragments, which are bivalent fragments in which two Fab fragments are linked by a disulfide bridge in a hinge region; (3) Fd fragments having two VH and CH1 domains; (4) Fv fragments having VL and VH domains of single antibody arms, (5) dAb fragments (Ward et al., "Binding Activities of a Repertoire of Single Immunoglobulin Variable Domains Secreted From Escherichia coli," Nature, 341:544-546(1989), which is incorporated herein by reference in its entirety) having VH domains; (6) isolated complementarity determining regions (CDRs), and (7) single-chain Fvs (scFvs), which are, for example, derived from a scFV-library. Although the two domains VL and VH of the Fv fragments are encoded by independent genes, they can be joined using a recombination method by synthetic linkers, which allow them to be prepared as single proteins in which the VL and VH regions are paired to form monovalent molecules (called single-chain Fvs (scFvs)) (see, for example, Huston et al., "Protein Engineering of Antibody Binding Sites: Recovery of Specific Activity in an Anti-Digoxin Single-Chain Fv Analogue Produced in Escherichia coli," Proc. Natl. Acad. Sci. USA, 85:5879-5883(1988)). These antibody fragments are obtained using conventional techniques known to those skilled in the art, and their functions are evaluated in the same way as that for the complete antibodies. In the present application, the term "antigen-binding fragment" further comprises ligands that specifically bind to antigens, in particular cell surface receptor molecular antigens. The ligands are generally polypeptides or compounds that can bind to receptor proteins (for example, the antigens) with high affinity and specificity to induce a functional response. For example, the ligands may comprise CD40L as a ligand of CD40, CD137L as a ligand of CD137, CD80 as a ligand of CD28, and/or OX-40L as a ligand of OX-40.

In the present application, the term "immune checkpoint" generally refers to a group of molecules on the cell surfaces of CD4⁺ and CD8⁺ T cells. These molecules can effectively act as a "brake" to down-regulate or suppress the anti-tumor immune response. These suppressive molecules can be suppressed by suppressing DNAs, RNAs or protein levels.

In the present application, the term "co-stimulatory receptor" generally refers to a class of receptors, which are expressed on the surface of immune cells, such as T cells and on which the activation of immune responses are dependent. On the premise that an antigen-presenting cell exists and a first signal has been provided, the costimulatory receptor turns on a costimulatory signal by binding to its corresponding activated antibody, ligand or antigen-binding fragment, thereby completely activating an immune effector cell. The costimulatory receptor-mediated activation of signaling pathways generally plays a key role in the effective occurrence of immune responses. Common costimulatory receptors comprise but are not limited to CD28, CD40, CD137 and OX-40.

In the present application, the term "tumor necrosis factor (TNF)" generally refers to a TNF protein, and is not a superfamily of TNF ligands. The Genbank Accession No. of human TNFα is NP_000585.2. The term "TNFα" encompasses pro-TNFα which is the precursor form of TNFα, fulllength TNFα, and any form of TNFα produced in an intracellular process. This term also encompasses naturally-occurring and non-naturally-occurring variants of TNFα, such as splice variants, allelic variants, and isoforms. TNFα can bind to two receptors, namely, TNFR1 (a type 1 TNF receptor; CD120a; p55/60) and TNFR2 (a type 2 TNF receptor; CD120b; p75/80). TNFα acts as a pro-inflammatory cytokine, for example in neuroinflammation.

In the present application, the term "tumor-infiltrating lymphocytes" generally refers to infiltrating lymphocytes isolated from tumor tissues. Immunohistochemical staining show that the tumor-infiltrating lymphocytes may comprise CD3⁺ T cells, CD20⁺, CD79α+B cells, plasma cells and CD56⁺ NK cells; and they may also comprise T cells, few B cells, NK cells, macrophages and dendritic cells. The tumor-infiltrating lymphocytes can produce highly efficient and highly specific anti-tumor killing effects directly by means of cytotoxic T lymphocytes and secretory cytokines. In the present application, the tumor-infiltrating lymphocytes may comprise mononuclear leukocytes, which leave the bloodstream and migrate to a tumor. The tumor-infiltrating lymphocytes may be selected from the group consisting of: T cells, B cells, natural killer cells, and natural killer T cells.

In the present application, the term "seed cells" generally refers to tumor-infiltrating lymphocytes cultured from tumor tissues using any suitable medium including the medium of the present application. These cells can act as a starting cell population for further expansion in the subsequent culture, or as a cell population for end uses, such as a cell population for infusion therapy to individual subjects. The seed cells can be obtained from tumor tissues undergoing any different culture duration, and the culture duration of the seed cells can be determined according to the subsequent use of the seed cells, and is not limited to a specific culture duration.

In the present application, the term "IL-15" generally refers to pro-inflammatory cytokines, also called "IL-15 antigen" and "interleukin 15", which act as effective growth, survival and activation factors for T cells (in particular intestinal intraepithelial lymphocytes (IEL)) and natural killer (NK) cells. It has been proved that the expression of IL-15 increases in a variety of inflammatory diseases, including CD, rheumatoid arthritis (RA) and psoriasis (Malamut et al., 2010). IL-15 is considered as a central regulating factor of CD immunopathogenesis and a non-redundant driving factor of lymphoma formation in RCD.

In the present application, the term "IL-2" generally refers to a lymphokine-derived protein which is produced from normal peripheral blood lymphocytes and exist in vivo at a low concentration. Initially, Morgan et al. (1976, Science 193: 1007-1008) described IL-2, which was originally called T cell growth factor, since it was capable of inducing the proliferation of stimulated T lymphocytes. This term also comprises modified (for example glycosylated, acetylated, phosphorylated, etc.) proteins of IL-2 after expression.

In the present application, the term "IL-7" generally refers to a protein encoded by an IL-7 gene. IL-7 is a hematopoietic growth factor secreted by stromal cells in bone marrow and thymus. It can be produced by keratinocytes, dendritic cells, hepatocytes, neurons and epithelial cells. IL-7 can be involved in an effect on T cells and B cells.

In the present application, the term "PD-1" generally refers to an immunosuppressive receptor belonging to a CD28 family. PD-1 is mainly expressed in vivo on previously activated T cells and binds to two ligands, namely, PD-1 and PD-2. A complete hPD-1 sequence can be found in GenBank under Accession No. U64863.

In the present application, the term "LYC-55716" generally refers to a RORγ activator. Its CAS NO. is 2055536-64-4.

In the present application, the term "GNE-1858" generally refers to an HPK1 inhibitor with CAS No. T11438.

In the present application, the term "TWS119" generally refers to a GSK-3 inhibitor with CAS No. 601514-19-6.

In the present application, the term "RRX-001" generally refers to an epigenetic modulator with radiosensitization activity. The RRX-001 can down-regulate the CD47/SIRPα axis and repolarize TAM and other immunosuppressive cells in a tumor microenvironment into an immunostimulatory phenotype. Its CAS NO. is 925206-65-1.

In the present application, the term "CNI-1493" generally refers to a compound capable of preventing the production of inflammatory cytokines (including TNF), and its CAS No. is 164301-51-3.

### DETAILED DESCRIPTION OF THE INVENTION

In one aspect, the present application provides a seed cell medium for tumor-infiltrating lymphocytes, comprising the following components: a cell culture ingredient, a cytokine, and an immune checkpoint antibody or an antigen-binding fragment thereof, wherein the cytokine comprises IL-2, the immune checkpoint comprises: PD-1, LAG-3, TIGIT and/or CTLA-4, and the cell culture ingredient is a serum-containing medium or a serum-free medium.

In the present application, the cytokine may be a human cytokine.

In some embodiments, the immune checkpoint antibody or the antigen-binding fragment thereof may be a humanized and/or fully-human antibody or an antigen-binding fragment thereof.

For example, the IL-2 may have a concentration of about 3000 IU/mL or less (for example, about 2900 IU/mL or less, about 2800 IU/mL or less, about 2700 IU/mL or less, about 2600 IU/mL or less, about 2500 IU/mL or less, about 2400 IU/mL or less, about 2300 IU/mL or less, about 2200 IU/mL or less, about 2100 IU/mL or less, about 2000 IU/mL or less, about 1900 IU/mL or less, about 1800 IU/mL or less, about 1700 IU/mL or less, about 1600 IU/mL or less, about 1500 IU/mL or less, about 1400 IU/mL or less, about 1300 IU/mL or less, about 1100 IU/mL or less, about 1100 IU/mL or less, about 1000 IU/mL or less, about 900 IU/mL or less, about 800 IU/mL or less, about 700 IU/mL or less, about 600 IU/mL or less, about 500 IU/mL or less, about 400 IU/mL or less, or about 300 IU/mL or less or even below). For example, the IL-2 may have a concentration of about 2000 IU/mL or less, about 2500 IU/mL or less, or about 3000 IU/mL or less.

For example, the cytokine may comprise IL-7.

For example, the IL-7 may have a concentration of about 5 ng/mL to about 100 ng/mL (for example, about 5 ng/mL to about 95 ng/mL, about 5 ng/mL to about 90 ng/mL, about 5 ng/mL to about 85 ng/mL, about 5 ng/mL to about 80 ng/mL, about 5 ng/mL to about 75 ng/mL, about 5 ng/mL to about 70 ng/mL, about 5 ng/mL to about 65 ng/mL, about 5 ng/mL to about 60 ng/mL, about 5 ng/mL to about 55 ng/mL, about 5 ng/mL to about 50 ng/mL, about 5 ng/mL to about 45 ng/mL, about 5 ng/mL to about 40 ng/mL, about 5 ng/mL to about 35 ng/mL, about 5 ng/mL to about 30 ng/mL, about 5 ng/mL to about 25 ng/mL, about 5 ng/mL to about 20 ng/mL, or about 5 ng/mL to about 10 ng/mL). For example, the IL-7 may have a concentration of about 200 U/mL to about 1000 U/mL (for example, at least about 200 U/mL, at least about 500 U/mL, or at least about 1000 U/mL). In the present application, the IL-7 may have a concentration of about 200-1000 IU/mL. For example, the concentration may be at least about 200 IU/mL, at least about 500 IU/mL, or at least about 1000 IU/mL.

For example, the cytokine may comprise IL-15.

For example, the IL-15 may have a concentration of about 5 ng/mL to about 100 ng/mL (for example, about 5 ng/mL to about 95 ng/mL, about 5 ng/mL to about 90 ng/mL, about 5 ng/mL to about 85 ng/mL, about 5 ng/mL to about 80 ng/mL, about 5 ng/mL to about 75 ng/mL, about 5 ng/mL to about 70 ng/mL, about 5 ng/mL to about 65 ng/mL, about 5 ng/mL to about 60 ng/mL, about 5 ng/mL to about 55 ng/mL, about 5 ng/mL to about 50 ng/mL, about 5 ng/mL to about 45 ng/mL, about 5 ng/mL to about 40 ng/mL, about 5 ng/mL to about 35 ng/mL, about 5 ng/mL to about 30 ng/mL, about 5 ng/mL to about 25 ng/mL, about 5 ng/mL to about 20 ng/mL, or about 5 ng/mL to about 10 ng/mL). For example, the IL-15 may have a concentration of about 200 U/mL to about 500 U/mL (for example, at least about 200 U/mL, at least about 250 U/mL, at least about 300 U/mL, or at least about 500 U/mL). In the present application, the IL-15 may have a concentration of about 200-500 IU/mL. For example, the concentration may be at least about 200 IU/mL, at least about 250 IU/mL, at least about 300 IU/mL, or at least about 500 IU/mL.

For example, the cytokine may comprise a tumor necrosis factor (TNF).

For example, the cytokine may comprise TNFα.

For example, the TNFα may have a concentration of about 10 pg to about 100pg/mL (for example, about 10 pg/mL to about 95pg/mL, about 10 pg/mL to about 90pg/mL, about 10 pg/mL to about 80pg/mL, about 10 pg/mL to about 70pg/mL, about 10 pg/mL to about 60pg/mL, about 10 pg/mL to about 50pg/mL, about 10 pg/mL to about 40pg/mL, about 15 pg/mL to about 40pg/mL, about 10 pg/mL to about 25pg/mL, about 15 pg/mL to about 25pg/mL, about 10 pg/mL to about 30pg/mL, about 10 pg/mL to about 20pg/mL, or about 10 pg/mL to about 15pg/mL). In the present application, the TNF-α may have a concentration of about 10-100 pg/mL. For example, the concentration may be about 10-100 pg/mL, about 15-100 pg/mL, about 20-100 pg/mL or about 25-100 pg/mL. For example, the concentration may be at least about 10 pg/mL, at least about 15 pg/mL, at least about 20 pg/mL, or at least about 25 pg/mL.

For example, the cytokine may comprise a colony stimulating factor.

For example, the cytokine comprises GM-CSF, G-CSF and/or M-CSF.

For example, the GM-CSF may have a concentration of about 200 U/mL to about 5000 U/mL (for example, about 200 U/mL to about 800 U/mL, about 200 U/mL to about 500 U/mL, about 300 U/mL to about 5000 U/mL, about 500 U/mL to about 5000 U/mL, about 1000 U/mL to about 5000 U/mL, about 1500 U/mL to about 5000 U/mL, about 2000 U/mL to about 5000 U/mL, about 2500 U/mL to about 5000 U/mL, about 3000 U/mL to about 5000 U/mL, about 3500 U/mL to about 5000 U/mL, about 4000 U/mL to about 5000 U/mL, or about 45000 U/mL to about 5000 U/mL). For example, the concentration may be about 200 U/mL to about 1000 U/mL, about 200 U/mL to about 800 U/mL, or about 200 U/mL to about 500 U/mL. For example, the concentration may be at least about 200 U/mL, at least about 500 U/mL, or at least about 800 U/mL.

For example, the G-CSF may have a concentration of about 300 U/mL to about 1000 U/mL. For example, the G-CSF may have a concentration of about 500-1000 U/mL. For example, the concentration may be at least about 500 U/mL or at least about 1000 U/mL.

For example, the M-CSF may have a concentration of about 300 U/mL to about 800U/mL. For example, the M-CSF may have a concentration of about 300 U/mL to about 500 U/mL. For example, the concentration may be at least about 300 U/mL, at least about 500 U/mL, or at least about 800 U/mL.

For example, the cytokine may comprise an interferon.

For example, the cytokine may comprise IFN-γ, IFN-α and/or IFN-β.

For example, the IFN-γ may have a concentration of about 10ng/mL to about 1000ng/mL (for example, about 15 ng/mL to about 1000ng/mL, about 20 ng/mL to about 1000ng/mL, about 30 ng/mL to about 1000ng/mL, about 40 ng/mL to about 1000ng/mL, about 50 ng/mL to about 1000ng/mL, about 60 ng/mL to about 1000ng/mL, about 70 ng/mL to about 1000ng/mL, about 80 ng/mL to about 1000ng/mL, about 90 ng/mL to about 1000ng/mL, about 100 ng/mL to about 1000ng/mL, about 200 ng/mL to about 1000ng/mL, about 300 ng/mL to about 1000ng/mL, about 400 ng/mL to about 1000ng/mL, about 500 ng/mL to about 1000ng/mL, about 600 ng/mL to about 1000ng/mL, about 700 ng/mL to about 1000ng/mL, about 800 ng/mL to about 1000ng/mL, or about 900 ng/mL to about 1000ng/mL). For example, the IFN-γ may have a concentration of about 10 ng/mL to about 1000 ng/mL. For example, the IFN-γ may have a concentration of about 300 ng/mL to about 1000 ng/mL, about 300 ng/mL to about 800 ng/mL, or about 300 ng/mL to about 500 ng/mL. For example, the concentration may be at least about 300 ng/mL, at least about 500 ng/mL, at least about 800 ng/mL, or at least about 1000 ng/mL

For example, the IFN-α may have a concentration of about 500 U/mL to about 1000 U/mL. For example, the concentration may be at least about 500 ng/mL.

For example, the IFN-β may have a concentration of about 200 U/mL to about 500 U/mL. For example, the IFN-β may have a concentration of about 250 ng/mL to about 500 ng/mL. For example, the concentration may be at least about 200 ng/mL, at least about 250 ng/mL, or at least about 500 ng/mL.

For example, the cytokine may further comprise: IL-4, IL-1α, IL-1β, IL-6, IL-9, IL-18, IL-12, IL-21 and/or IL-10.

For example, the IL-4 may have a concentration of about 200 U/mL to about 500 U/mL. For example, the concentration may be at least about 200 IU/mL, or at least about 500 IU/mL.

For example, the IL-1α may have a concentration of about 200 U/mL to about 500 U/mL. For example, the concentration may be at least about 200 IU/mL, or at least about 500 IU/mL.

For example, the IL-Iβ may have a concentration of about 200 U/mL to about 500 U/mL. For example, the concentration may be at least about 200 IU/mL, or at least about 500 IU/mL.

For example, the IL-6 may have a concentration of about 200 U/mL to about 500 U/mL. For example, the concentration may be at least about 200 IU/mL, or at least about 500 IU/mL.

For example, the IL-9 may have a concentration of about 200 U/mL to about 500 U/mL. For example, the concentration may be at least about 200 IU/mL, or at least about 500 IU/mL.

For example, the IL-18 may have a concentration of about 200 U/mL to about 500 U/mL. For example, the concentration may be at least about 200 IU/mL, or at least about 500 IU/mL.

For example, the IL-12 may have a concentration of about 200 U/mL to about 1000 U/mL. For example, the IL-12 may have a concentration of about 200 U/mL to about 1000 U/mL, about 200 U/mL to about 500 U/mL, or about 300 U/mL to about 1000 U/mL. For example, the concentration may be at least about 200 IU/mL, at least about 250 IU/mL, at least about 300 IU/mL. or at least about 500 IU/mL.

For example, the IL-21 may have a concentration of about 200 U/mL to about 500 U/mL. For example, the IL-21 may have a concentration of about 250 U/mL to about 1000 U/mL. For example, the concentration may be at least about 200 U/mL, at least about 250 U/mL, or at least about 500 IU/mL.

For example, the IL-10 may have a concentration of about 200 U/mL to about 500 U/mL. For example, the concentration may be at least about 200 U/mL, at least about 250 U/mL, at least about 300 U/mL, or at least about 500 IU/mL.

For example, the immune checkpoint antibody or the antigen-binding fragment thereof may be a PD-1 antibody or an antigen-binding fragment thereof.

For example, the PD-1 antibody or the antigen-binding fragment thereof may be a human PD-1 antibody or an antigen-binding fragment thereof.

For example, the PD-1 antibody or the antigen-binding fragment thereof may have a concentration of about 1 µg/mL to about 100 µg/mL (for example, about 3 µg/mL to about 100 µg/mL, about 5 µg/mL to about 100 µg/mL, about 10 µg/mL to about 100 µg/mL, about 15 µg/mL to about 100 µg/mL, about 16 µg/mL to about 100 µg/mL, about 17 µg/mL to about 100 µg/mL, about 18 µg/mL to about 100 µg/mL, about 19 µg/mL to about 100 µg/mL, about 20 µg/mL to about 100 µg/mL, about 25 µg/mL to about 100 µg/mL, about 30 µg/mL to about 100 µg/mL, about 36 µg/mL to about 100 µg/mL, about 40 µg/mL to about 100 µg/mL, about 45 µg/mL to about 100 µg/mL, about 50 µg/mL to about 100 µg/mL, about 55 µg/mL to about 100 µg/mL, about 60 µg/mL to about 100 µg/mL, about 65 µg/mL to about 100 µg/mL, about 70 µg/mL to about 100 µg/mL, about 80 µg/mL to about 100 µg/mL, or about 90 µg/mL to about 100 µg/mL). In the present application, the PD-1 mAb may have a concentration of about 3 µg/mL to 100 µg/mL. For example, the concentration may be at least about 3 µg/mL, at least about 5 µg/mL, at least about 10 µg/mL, at least about 15 µg/mL, at least about 20 µg/mL, at least about 25 µg/mL, at least about 36 µg/mL, at least about 50 µg/mL, or at least about 100 µg/mL.

For example, the immune checkpoint antibody or the antigen-binding fragment thereof may be a human LAG-3 antibody or an antigen-binding fragment thereof.

For example, the immune checkpoint antibody or the antigen-binding fragment thereof may be an LAG-3 antibody or an antigen-binding fragment thereof, and the LAG-3 antibody or the antigen-binding fragment thereof has a concentration of about 3 µg/mL to about 10 µg/mL. For example, the concentration may be about 5 µg/mL to about 10 µg/mL. For example, the concentration may be at least about 3 µg/mL, at least about 5 µg/mL or at least about 10 µg/mL.

For example, the TIGIT antibody or the antigen-binding fragment thereof may be a human TIGIT antibody or an antigen-binding fragment thereof.

For example, the immune checkpoint antibody or the antigen-binding fragment thereof may be a TIGIT antibody or an antigen-binding fragment thereof, and the TIGIT antibody or the antigen-binding fragment thereof has a concentration of about 1 µg/mL to about 25 µg/mL. In the present application, the TIGIT mAb may have a concentration of about 2.5 µg/mL to 20 µg/mL. For example, the concentration may be about 5-20 µg/mL. For example, the concentration may be at least about 2.5 µg/mL, at least about 5 µg/mL or at least about 20 µg/mL.

For example, the immune checkpoint antibody or the antigen-binding fragment thereof may be a human CTLA-4 antibody or an antigen-binding fragment thereof.

For example, the immune checkpoint antibody or the antigen-binding fragment thereof may be a CTLA-4 antibody or an antigen-binding fragment thereof, and the CTLA-4 antibody or the antigen-binding fragment thereof has a concentration of about 1 µg/mL to about 100 µg/mL. In the present application, the CTLA-4 mAb may have a concentration of about 3-50 µg/mL, about 5-50 µg/mL or about 10-50 µg/mL. For example, the concentration may be at least about 3 µg/mL, at least about 5 µg/mL at least about 10 µg/mL, or at least about 50 µg/mL.

In the present application, the seed cell medium may further comprise a costimulatory receptor antibody or an antigen-binding fragment thereof, wherein the costimulatory receptor immune checkpoint antibody or the antigen-binding fragment thereof may comprise: a CD40 antibody or an antigen-binding fragment, an OX-40 antibody or an antigen-binding fragment thereof, a CD137 antibody or an antigen-binding fragment thereof, and/or a CD28 antibody thereof or an antigen-binding fragment thereof.

For example, the costimulatory antibody or the antigen-binding fragment thereof may comprise a CD137 antibody or an antigen-binding fragment thereof.

For example, the CD137 antibody or the antigen-binding fragment thereof may be a human CD137 antibody or an antigen-binding fragment thereof.

For example, the CD137 antibody or the antigen-binding fragment thereof may have a concentration of about 1 µg/mL to about 100 µg/mL (for example, about 2 µg/mL to about 100 µg/mL, about 3 µg/mL to about 100 µg/mL, about 3 µg/mL to about 20 µg/mL, about 4 µg/mL to about 100 µg/mL, about 5 µg/mL to about 100 µg/mL, about 6 µg/mL to about 100 µg/mL, about 7 µg/mL to about 100 µg/mL, about 8 µg/mL to about 100 µg/mL, about 9 µg/mL to about 100 µg/mL, about 10 µg/mL to about 100 µg/mL, about 12 µg/mL to about 100 µg/mL, about 20 µg/mL to about 100 µg/mL, about 10 µg/mL to about 90 µg/mL, about 10 µg/mL to about 80 µg/mL, about 10 µg/mL to about 70 µg/mL, about 10 µg/mL to about 50 µg/mL, about 10 µg/mL to about 20 µg/mL, or about 3 µg/mL to about 12 µg/mL). In the present application, the CD137 mAb may have a concentration of about 3 µg/mL to 20 µg/mL. For example, the concentration may be at least about 3 µg/mL, at least about 5 µg/mL, at least about 10 µg/mL, at least about 12 µg/mL, or at least about 20 µg/mL.

For example, the costimulatory antibody or the antigen-binding fragment thereof may comprise an OX40 antibody or an antigen-binding fragment thereof.

For example, the OX-40 antibody or the antigen-binding fragment thereof may be a human OX-40 antibody or an antigen-binding fragment thereof.

For example, the OX-40 antibody or the antigen-binding fragment thereof has a concentration of about 3 µg/mL to about 10 µg/mL. In the present application, the OX-40 mAb may have a concentration of about 5 µg/mL to 10 µg/mL. For example, the concentration may be at least about 3 µg/mL, at least about 5 µg/mL or at least about 10 µg/mL.

For example, the costimulatory antibody or the antigen-binding fragment thereof may comprise a CD28 antibody or an antigen-binding fragment thereof.

For example, the immune checkpoint antibody or the antigen-binding fragment thereof may comprise a CD28 antibody or an antigen-binding fragment thereof.

For example, the CD28 antibody or the antigen-binding fragment thereof may be a human CD28 antibody or an antigen-binding fragment thereof.

For example, the CD28 antibody or the antigen-binding fragment thereof may have a concentration of about 1 µg/mL to about 10 µg/mL. (For example, the concentration may be about 2 µg/mL to about 10 µg/mL, about 3 µg/mL to about 10 µg/mL, about 3 µg/mL to about 5 µg/mL, about 4 µg/mL to about 10 µg/mL, about 5 µg/mL to about 10 µg/mL, about 6 µg/mL to about 10µg/mL, about 7 µg/mL to about 10 µg/mL or about 8 ng/mL to about 10µg/mL.) In the present application, the CD28 mAb may have a concentration of about 3 µg/mL to 10 µg/mL. For example, the concentration may be at least about 3 µg/mL, at least about 5 µg/mL or at least about 10 µg/mL.

For example, the costimulatory antibody or the antigen-binding fragment thereof may comprise a CD40 antibody or an antigen-binding fragment thereof.

For example, the CD40 antibody or the antigen-binding fragment thereof may be a human CD40 antibody or an antigen-binding fragment thereof.

For example, the CD40 antibody or the antigen-binding fragment thereof may have a concentration of about 5 µg/mL to about 10 µg/mL. In the present application, the CD40 mAb may have a concentration of about 6-10 µg/mL, or about 5-8 µg/mL. For example, the concentration may be at least about 5 µg/mL.

For example, the serum medium may comprise a serum.

For example, the serum may comprise a human AB serum.

For example, the serum may have a concentration of about 1% to about 10% (v/v) (for example, about 1.5% to about 10%, about 2% to about 10%, about 3% to about 10%, about 4% to about 10%, about 5% to about 10%, about 1% to about 9%, about 1% to about 8%, about 1% to about 7%, or about 1% to about 6%).

For example, the serum medium may comprise an AIM-V medium.

For example, the serum-free medium may comprise an X-VIVO medium or a CTS^{™}OpTmizer^{™} medium.

For example, the cell culture ingredient may comprise antibiotics.

For example, the cell culture ingredient may comprise a penicillin-streptomycin (PS) mixed solution.

For example, the PS mixed solution may have a concentration of about 1 U/mL to about 200 U/mL (for example, about 1 U/mL to about 5 U/mL, about 1 U/mL to about 10 U/mL, about 1 U/mL to about 20 U/mL, about 1 U/mL to about 30 U/mL, about 1 U/mL to about 40 U/mL, about 1 U/mL to about 50 U/mL, about 1 U/mL to about 60 U/mL, about 1 U/mL to about 70 U/mL, about 1 U/mL to about 80 U/mL, about 1 U/mL to about 90 U/mL, about 1 U/mL to about 100 U/mL, about 1 U/mL to about 120 U/mL, about 1 U/mL to about 140 U/mL, about 1 U/mL to about 160 U/mL, about 1 U/mL to about 180 U/mL, or about 1 U/mL to about 200 U/mL). For example, the PS mixed solution may have a concentration of about 100 U/mL.

For example, the seed cell medium may further comprise an M2 macrophage inhibitor, which may comprise RRx001 and/or CNI-1493.

For example, the M2 macrophage inhibitor may have a concentration of about 0.1 µM to about 100 µM (for example, about 0.5 µM to about 100 µM, about 1 µM to about 100 µM, about 2 µM to about 100 µM, about 2.5 µM to about 100 µM, about 3 µM to about 100 µM, about 2 µM to about 3 µM, about 5 µM to about 100 µM, about 10 µM to about 100 µM, about 20 µM to about 100 µM, about 0.1 µM to about 90 µM, about 0.5 µM to about 90 µM, about 1 µM to about 90 µM, about 5 µM to about 90 µM, or about 0.1 µM to about 80 µM).

For example, the seed cell medium may further comprise a regulatory T cell (Treg) inhibitor, which may comprise CAL-101, dasatinib, imatinib and/or panobinostat.

For example, the regulatory T cell inhibitor may have a concentration of about 0.1 µM to about 100µM (for example, about 0.5 µM to about 100µM, about 1 µM to about 100µM, about 5 µM to about 100µM, about 10 µM to about 100µM, about 20 µM to about 100µM, about 0.1 µM to about 90µM, about 0.5 µM to about 90µM, about 1 µM to about 90µM, about 5 µM to about 90µM, or about 0.1 µM to about 80µM). For example, the regulatory T cell inhibitor may have a concentration of about 1 µM to about 5 µM, about 1 µM to about 3 µM, about 1.5 µM to about 5 µM, about 2 µM to about 5 µM, about 2.5 µM to about 5 µM, or about 3 µM to about 5 µM.

For example, the seed cell medium may further comprise a myeloid-derived suppressor cell (MDSC) inhibitor, and the myeloid-derived suppressor cell inhibitor may comprise AG490, decitabine, sunitinib and/or BBI608.

For example, the bone myeloid-derived suppressor cell inhibitor may have a concentration of about 0.1 µg/mL to about 100 µg/mL (for example, about 0.5 µg/mL to about 100 µg/mL, about 1 µg/mL to about 100 µg/mL, about 2 µg/mL to about 100 µg/mL, about 5 µg/mL to about 100 µg/mL, about 6 µg/mL to about 100 µg/mL, about 7 µg/mL to about 100 µg/mL, about 8 µg/mL to about 100 µg/mL, about 9 µg/mL to about 100 µg/mL, about 10 µg/mL to about 100 µg/mL, about 20 µg/mL to about 100 µg/mL, about 10 µg/mL to about 90 µg/mL, about 10 µg/mL to about 80 µg/mL, about 10 µg/mL to about 70 µg/mL, or about 10 µg/mL to about 50 µg/mL). For example, the myeloid-derived suppressor cell agent may have a concentration of about 1 µg/mL to about 3 µg/mL, or about 1.5 µg/mL to about 3 µg/mL.

In the present application, the seed medium may comprise at least one, at least two, and at least three of the regulatory T cell (Treg) inhibitory, the myeloid-derived suppressor cell inhibitor, and the M2 macrophage inhibitor.

In the present application , the seed cell medium may further comprise a T cell activator, and the T cell activator may comprise LYC-55716, GNE-1858 and/or methylene blue.

For example, the T cell activator may have a concentration of about 1 µM to about 10 µM. For example, the concentration may be about 1 µM to about 9 µM, about 1 µM - about 8µM, about 1 µM to about 7µM, about 1 µM to about 6µM, about 2 µM to about 10µM, about 3 µM to about 10µM, about 4 µM to about 10µM or about 5 µM to about 10µM.

In the present application, the seed cell medium may further comprise a T cell differentiation inhibitor, and the T cell differentiation inhibitor may comprise TWS119.

For example, the T cell differentiation inhibitor may have a concentration of about 1 µM to about 10 µM. For example, the concentration may be about 1 µM to about 9 µM, about 1 µM - about 8µM, about 1 µM to about 7µM, about 1 µM to about 6µM, about 2 µM to about 10µM, about 3 µM to about 10µM, about 4 µM to about 10µM or about 5µM to about 10µM.

In the present application, the RRX-001 may have a concentration of about 0.1 µM to about 100 µM. For example, the concentration may be at least about 3 µM.

In the present application, the sunitinib may have a concentration of about 1.5 µM to about 3 µM. For example, the concentration may be at least about 1.5 µM or at least about 3 µM.

In the present application, the CNI-1493 may have a concentration of about 2 µM to about 3 µM. For example, the concentration may be at least about 2µM, at least about 2.5 µM, or at least about 3 µM.

In the present application, the imatinib may have a concentration of about 2 µM to about 5 µM. For example, the concentration may be at least about 2 µM or at least about 5 µM.

In the present application, the CAL -101 may have a concentration of about 1.5 µM to about 5 µM. For example, the concentration may be at least about 1.5 µM or at least about 5 µM.

In the present application, the dasatinib may have a concentration of about 2 µM to about 3 µM. For example, the concentration may be at least about 2µM, at least about 2.5 µM, or at least about 3 µM.

In the present application, the LYC-55716 may have a concentration of about 1 µM to about 10 µM. For example, the concentration may be at least about 1 µM or at least about 10 µM.

In the present application, the GNE-1858 may have a concentration of about 4 nM to about 5 nM. For example, the concentration may be at least about 4 nM, at least about 4.5 nM, or at least about 5 nM.

In the present application, the methylene blue may have a concentration of about 1 µM to about 2 µM. For example, the concentration may be at least about 1 µM, at least about 1.5 µM, or at least about 2 µM.

In the present application, the TWS119 may have a concentration of about 1 µM to about 10 µM. For example, the concentration may be at least about 1 µM, at least about 5 µM, or at least about 10 µM.

In the present application, the seed medium may comprise IL-2, IL-7 and PD-1 antibodies or antigen-binding fragments thereof, TNFα, a human serum, a serum-free medium X-VIVO 15, and a penicillin-streptomycin (PS) dual-antibiotics mixed solution.

For example, the seed medium may comprise at least about 3000 U/mL IL-2, at least about 1000 U/mL IL-7, at least about 100 µg/mL PD-1 antibody or the antigen-binding fragment thereof, at least about 10 pg/mL TNFα, at least about 5% human serum, a serum-free medium X-VIVO 15, and the 100 U/mL PS double-antibiotics mixed solution.

In the present application, the seed medium may comprise IL-2, IL-9, IL-15 and PD-1 antibodies or antigen-binding fragments thereof, a CTLA-4 antibody or an antigen-binding fragment thereof, a human serum, a serum-free medium X-VIVO 15, and a PS dual-antibiotics mixed solution.

For example, the seed medium may comprise at least about 2000 U/mL IL-2, at least about 500 U/mL IL-9, at least about 500 U/mL IL-15, at least about 50 µg/mL CTLA-4 antibody or an antigen-binding fragment thereof, at least about 50 µg/mL PD-1 antibody or an antigen-binding fragment thereof, at least about 3% human serum, a serum-free medium AIM-V, and the 100 U/mL PS dual-antibiotics mixed solution.

In the present application, the seed medium may comprise IL-2, IL-7, G-CSF and PD-1 antibodies or antigen-binding fragments thereof, TNFα, a human serum, a serum-free medium X-VIVO 15, and a PS dual-antibiotics mixed solution.

For example, the seed medium may comprise at least about 2000 U/mL IL-2, at least about 500 U/mL IL-7, at least about 1000 U/mL G-CSF, at least about 36 µg/mL PD-1 antibody or the antigen-binding fragment thereof, at least about 20 pg/mL TNFα, at least about 3% human serum, the serum-free medium AIM-V, and the 100 U/mL PS dual-antibiotics mixed solution.

In the present application, the seed medium may comprise IL-2, IL-12, IL-21, GM-CSF and LAG3 antibodies or antigen-binding fragments thereof, a CTLA-4 antibody or an antigen-binding fragment thereof, a human serum, a serum-free medium X-VIVO 15, and a PS dual-antibiotics mixed solution.

For example, the seed medium may comprise at least about 3000 U/mL IL-2, at least about 200 U/mL IL-12, at least about 500 U/mL IL-21, at least about 800 U/mL GM-CSF, at least about 10 µg/mL LAG-3 antibody or the antigen-binding fragment thereof, at least about 10 µg/mL CTLA-4 antibody or the antigen-binding fragment thereof, at least about 5% human serum, the serum-free medium CTS^{™}OpTmizer^{™}, and the 100 U/mL PS double-antibiotics mixed solution.

In the present application, the seed medium may comprise IL-1β, IL-2, IL-6, IL-21, IFN-γ and TIGIT antibodies or antigen-binding fragments thereof, a PD-1 antibody or an antigen-binding fragment thereof, TNFα, a human serum, a serum-free medium X-VIVO 15, and a PS dual-antibiotics mixed solution.

For example, the seed medium may comprise at least about 500 U/mL IL-1β, at least about 2500 U/mL IL-2, at least about 200 U/mL IL-6, at least about 200 U/mL IL-21, at least about 1000 U/mL IFN-γ, at least about 20 µg/mL TIGIT antibody or the antigen-binding fragment thereof, at least about 50 µg/mL PD-1 antibody or the antigen-binding fragment thereof, at least about 25 pg/mL TNFα, at least about 5% human serum, the serum-free medium X-VIVO 15, and the 100 U/mL PS dual-antibiotics mixed solution.

In the present application, the seed medium may comprise IL-1α, IL-2, IL-9, IL-18, IFN-α, IFN-β and LAG-3 antibodies or antigen-binding fragments thereof, a PD-1 antibody or an antigen-binding fragment thereof, TNFα, a human serum, a serum-free medium X-VIVO 15, and a PS dual-antibiotics mixed solution.

For example, the seed medium may comprise at least about 500 U/mL IL-1α, at least about 2000 U/mL IL-2, at least about 500 U/mL IL-9, at least about 200 U/mL IL-18, at least about 500 U/mL IFN-α, at least about 500 U/mL IFN-β, at least about 5 µg/mL LAG-3 antibody or the antigen-binding fragment thereof, at least about 25 µg/mL PD-1 antibody or the antigen-binding fragment thereof, at least about 25 pg/mL TNFα, at least about 5% human serum, the serum-free medium X-VIVO 15, and the 100 U/mLPS dual-antibiotics mixed solution.

In the present application, the seed medium may comprise IL-2, IL-4, IL-10, IL-21 and CD137 antibodies or antigen-binding fragments thereof, a LAG-3 antibody or an antigen-binding fragment thereof, a PD-1 antibody or an antigen-binding fragment thereof, TNFα, a human serum, a serum-free medium X-VIVO 15, and a PS dual-antibiotics mixed solution.

For example, the seed medium may comprise at least about 3000 U/mL IL-2, at least about 200 U/mL IL-4, at least about 200 U/mL IL-10, at least about 250 U/mL IL-21, at least about 10 µg/mL CD137 antibody or the antigen-binding fragment thereof, at least about 3 µg/mL LAG-3 antibody or the antigen-binding fragment thereof, at least about 20 µg/mL PD-1 antibody or the antigen-binding fragment thereof, at least about 10 pg/mL TNFα, at least about 5% human serum, the serum-free medium X-VIVO 15, and 100 U/mLPS dual-antibiotics mixed solution.

In the present application, the seed medium may comprise IL-2, IL-6, IL-12, IL-21 and CD137 antibodies or antigen-binding fragments thereof, a CD28 antibody or an antigen-binding fragment thereof, a PD-1 antibody or an antigen-binding fragment thereof, TNFα, a human serum, a serum-free medium X-VIVO 15, and a PS dual-antibiotics mixed solution.

For example, the seed medium may comprise at least about 3000 U/mL IL-2, at least about 500 U/mL IL-6, at least about 500 U/mL IL-12, at least about 200 U/mL IL-21, at least about 20 µg/mL CD137 antibody or the antigen-binding fragment thereof, at least about 10 µg/mL CD28 antibody or the antigen-binding fragment thereof, at least about 10 µg/mL PD-1 antibody or the antigen-binding fragment thereof, at least about 10 pg/mL TNFα, at least about 3% human serum, the serum-free medium CTS^{™}OpTmizer^{™}, and the 100 U/mL PS dual-antibiotics mixed solution.

In the present application, the seed medium may comprise IL-1β, IL-2, IL-7, IL-21, G-CSF, GM-CSF, IFN-γ and LAG-3 antibodies or antigen-binding fragments thereof, a PD-1 antibody or an antigen-binding fragment thereof, TNFα, a human serum, a serum-free medium X-VIVO 15, and a PS dual-antibiotics mixed solution.

For example, the seed medium may comprise at least about 200 U/mL IL-1β, at least about 3000 U/mL IL-2, at least about 200 U/mL IL-7, at least about 200 U/mL IL-21, at least about 500 U/mL G-CSF, at least about 500 U/mL GM-CSF, at least about 1000 U/mL IFN-γ, at least about 5 µg/mL LAG-3 antibody or the antigen-binding fragment thereof, at least about 3 µg/mL PD-1 antibody or the antigen-binding fragment thereof, at least about 15 pg/mL TNFα, at least about 5% human serum, the serum-free medium CTS^{™}OpTmizer^{™}, and the 100 U/mLPS dual-antibiotics mixed solution.

In the present application, the seed medium may comprise IL-2, IL-4, IL-12, GM-CSF, M-CSF, IFN-β, IFN-γ and TIGIT antibodies or antigen-binding fragments thereof, a CTLA-4 antibody or an antigen-binding fragment thereof, a human serum, a serum-free medium X-VIVO 15, and a PS dual-antibiotics mixed solution.

For example, the seed medium may comprise at least about 2500 U/mL IL-2, at least about 500 U/mL IL-4, at least about 500 U/mL IL-12, at least about 500 U/mL GM-CSF, at least about 300 U/mL M-CSF, at least about 200 U/mL IFN-β, at least about 800 U/mL IFN-γ, at least about 5 µg/mL TIGIT antibody or the antigen-binding fragment thereof, at least about 10 µg/mL CTLA-4 antibody or the antigen-binding fragment thereof, at least about 5% human serum, the serum-free medium X-VIVO 15, and the 100 U/mLPS dual-antibiotics mixed solution.

In the present application, the seed medium may comprise IL-2, IL-7, IL-15, IL-2, IL-7, IL-15, GM-CSF and CD137 antibodies or antigen-binding fragments thereof, a PD-1 antibody or an antigen-binding fragment thereof, a TNFα human serum, a serum-free medium X-VIVO 15, and a PS dual-antibiotics mixed solution.

For example, the seed medium may comprise at least about 3000 U/mL IL-2, at least about 200 U/mL IL-7, at least about 200 U/mL IL-15, at least about 800 U/mL GM-CSF, at least about 10 µg/mL CD137 antibody or the antigen-binding fragment thereof, at least about 20 µg/mL PD-1 antibody or the antigen-binding fragment thereof, at least about 20 pg/mL TNFα, at least about 5% human serum, the serum-free medium X-VIVO 15, and the 100 U/mLPS dual-antibiotics mixed solution.

In the present application, the seed medium may comprise IL-2, IL-4, IL-10, IL-10, G-CSF, M-CSF and CD137 antibodies or antigen-binding fragments thereof, a CD28 antibody or an antigen-binding fragment thereof, an OX-40 antibody or an antigen-binding fragment thereof, a PD-1 antibody or an antigen-binding fragment thereof, a human serum, a serum-free medium X-VIVO 15, and a PS dual-antibiotics mixed solution.

For example, the seed medium may comprise at least about 2500 U/mL IL-2, at least about 500 U/mL IL-4, at least about 500 U/mL IL-10, at least about 250 U/mL IL-10, at least about 500 U/mL G-CSF, at least about 300 U/mL M-CSF, at least about 5 µg/mL CD137 antibody or the antigen-binding fragment thereof, at least about 3 µg/mL CD28 antibody or the antigen-binding fragment thereof, at least about 10 µg/mL OX-40 antibody or the antigen-binding fragment thereof, at least about 10 µg/mL PD-1 antibody or the antigen-binding fragment thereof, at least about 5% human serum, the serum-free medium AIM-V, and the 100 U/mLPS dual-antibiotics mixed solution.

In the present application, the seed medium may comprise IL-2, IL-7, IL-21, IFN-α, IFN-γ and CD137 antibodies or antigen-binding fragments thereof, a CD28 antibody or an antigen-binding fragment thereof, an OX-40 antibody or an antigen-binding fragment thereof, at least approximately an LAG-3 antibody or an antigen-binding fragment thereof, a CTLA-4 antibody or an antigen-binding fragment thereof, TNFα, a human serum, a serum-free medium X-VIVO 15, and a PS dual-antibiotics mixed solution.

For example, the seed medium may comprise at least about 3000 U/mL IL-2, at least about 200 U/mL IL-7, at least about 500 U/mL IL-21, at least about 500 U/mL IFN-α, at least about 800 U/mL IFN-γ, at least about 5 µg/mL CD137 antibody or the antigen-binding fragment thereof, at least about 3 µg/mL CD28 antibody or the antigen-binding fragment thereof, at least about 5 µg/mL OX-40 antibody or the antigen-binding fragment thereof, at least about 3 µg/mL LAG-3 antibody or the antigen-binding fragment thereof, at least about 5 µg/mL CTLA-4 antibody or the antigen-binding fragment thereof, at least about 10 pg/mL TNFα, at least about 5% human serum, the serum-free medium X-VIVO 15, and the 100 U/mL PS dual-antibiotics mixed solution.

In the present application, the seed medium may comprise IL-2, IL-7, IL-15, IFN-β, IFN-γ and CD137 antibodies or antigen-binding fragments thereof, a CD40 antibody or an antigen-binding fragment thereof, an OX-40 antibody or an antigen-binding fragment thereof, a TIGIT antibody or an antigen-binding fragment thereof, a PD-1 antibody or an antigen-binding fragment thereof, a human serum, a serum-free medium X-VIVO 15, and a PS dual-antibiotics mixed solution.

For example, the seed medium may comprise at least about 3000 U/mL IL-2, at least about 500 U/mL IL-7, at least about 500 U/mL IL-15, at least about 250 U/mL IFN-β, at least about 1000 U/mL IFN-γ, at least about 5 µg/mL CD137 antibody or the antigen-binding fragment thereof, at least about 5 µg/mL CD40 antibody or the antigen-binding fragment thereof, at least about 5 µg/mL OX-40 antibody or the antigen-binding fragment thereof, at least about 5 µg/mL TIGIT antibody or the antigen-binding fragment thereof, at least about 15 µg/mL PD-1 antibody or the antigen-binding fragment thereof, at least about 5% human serum, the serum-free medium X-VIVO 15, and the 100 U/mL PS dual-antibiotics mixed solution.

In the present application, the seed medium may comprise IL-2, IL-7, IL-15, GM-CSF, IFN-γ and CD137 antibodies or antigen-binding fragments thereof, a CD28 antibody or an antigen-binding fragment thereof, a PD-1 antibody or an antigen-binding fragment thereof, TNFα, at least about 5% human serum, a serum-free medium CTS^{™}OpTmizer^{™}, and a PS dual-antibiotics mixed solution.

For example, the seed medium may comprise at least about 3000 U/mL IL-2, at least about 200 U/mL IL-7, at least about 200 U/mL IL-15, at least about 500 U/mL GM-CSF, at least about 1000 U/mL IFN-γ, at least about 3 µg/mL CD137 antibody or the antigen-binding fragment thereof, at least about 3 µg/mL CD28 antibody or the antigen-binding fragment thereof, at least about 3 µg/mL PD-1 antibody or the antigen-binding fragment thereof, at least about 10 pg/mL TNFα, at least about 5% human serum, the serum-free medium CTS^{™}OpTmizer^{™}, and the 100 U/mL PS dual-antibiotics mixed solution.

In the present application, the seed medium may comprise IL-2, IL-7, IL-12, G-CSF, GM-CSF, IFN-α, IFN-γ and CD28 antibodies or antigen-binding fragments thereof, a CD40 antibody or an antigen-binding fragment thereof, a TIGIT antibody or an antigen-binding fragment thereof, a PD-1 antibody or an antigen-binding fragment thereof, TNFα, a human serum, a serum-free medium X-VIVO 15, and a PS dual-antibiotics mixed solution.

For example, the seed medium may comprise at least about 3000 U/mL IL-2, at least about 200 U/mL IL-7, at least about 300 U/mL IL-12, at least about 300 U/mL G-CSF, at least about 200 U/mL GM-CSF, at least about 500 U/mL IFN-α, at least about 500 U/mL IFN-γ, at least about 3 µg/mL CD28 antibody or the antigen-binding fragment thereof, at least about 5 µg/mL CD40 antibody or the antigen-binding fragment thereof, at least about 2.5 µg/mL TIGIT antibody or the antigen-binding fragment thereof, at least about 3 µg/mL PD-1 antibody or the antigen-binding fragment thereof, at least about 10 pg/mL TNFα, at least about 5% human serum, the serum-free medium X-VIVO 15, and the 100 U/mL PS dual-antibiotics mixed solution.

In the present application, the seed medium may comprise IL-2, IL-7 and PD-1 antibodies or antigen-binding fragments thereof, RRx-001, CAL-101, a human serum, a serum-free medium CTS^{™}OpTmizer^{™}, and a PS dual-antibiotics mixed solution.

For example, the seed medium may comprise at least about 3000 U/mL IL-2, at least about 1000 U/mL IL-7, at least about 50 µg/mL PD-1 antibody or the antigen-binding fragment thereof, at least about 3 µM RRx-001, at least about 1.5 µM CAL-101, at least about 5% human serum, the serum-free medium CTS^{™}OpTmizer^{™}, and the 100 U/mL PS dual-antibiotics mixed solution.

In the present application, the seed medium may comprise IL-2, IL-10, IL-18 and LAG-3 antibodies or antigen-binding fragments thereof, a PD-1 antibody or an antigen-binding fragment thereof, sunitinib, imatinib, a human serum, a serum-free medium X-VIVO 15, and a PS dual-antibiotics mixed solution.

For example, the seed medium may comprise at least about 2500 U/mL IL-2, at least about 200 U/mL IL-10, at least about 200 U/mLIL-18, at least about 10 µg/mL LAG-3 antibody or the antigen-binding fragment thereof, at least about 5 µg/mL PD-1 antibody or the antigen-binding fragment thereof, at least about 3 µM sunitinib, at least about 5 µM imatinib, at least about 5% human serum, the serum-free medium X-VIVO 15, and the 100 U/mLPS dual-antibiotics mixed solution.

In the present application, the seed medium may comprise IL-2, IL-1β, IL-6, GM-CSF, M-CSF, CNI-1493, a human serum, a serum-free medium X-VIVO 15, and a PS dual-antibiotics mixed solution.

For example, the seed medium may comprise at least about 3000 U/mL IL-2, at least about 200 U/mL IL-1β, at least about 500 U/mL IL-6, at least about 500 U/mL GM-CSF, at least about 500 U/mL M-CSF, at least about 5 µM CNI-1493, at least about 5% human serum, the serum-free medium X-VIVO 15, and the 100 U/mLPS ual-antibiotics mixed solution.

In the present application, the seed medium may comprise IL-2, IL-9, IL-10, IL-21, GM-CSF and OX40 antibodies or antigen-binding fragments thereof, a PD-1 antibody or an antigen-binding fragment thereof, sunitinib, a human serum, a serum-free medium X-VIVO 15, and a PS double-antibiotics mixed solution.

For example, the seed medium may comprise at least about 2000 U/mL IL-2, at least about 200 U/mL IL-9, at least about 200 U/mL IL-10, at least about 500 U/mL IL-21, at least about 500 U/mL GM-CSF, at least about 5 µg/mL OX40 antibody or the antigen-binding fragment thereof, at least about 36 µg/mL PD-1 antibody or the antigen-binding fragment thereof, at least about 1.5 µM sunitinib, at least about 5% human serum, the serum-free medium X-VIVO 15, and the 100 U/mLPS dual-antibiotics mixed solution.

In the present application, the seed medium may comprise IL-2, IL-6, IL-12 and CD28 antibodies or antigen-binding fragments thereof, an OX-40 antibody or an antigen-binding fragment thereof, a CTLA-4 antibody or an antigen-binding fragment thereof, TNFα, imatinib, a human serum, a serum-free medium CTS^{™}OpTmizer^{™}, and a PS dual-antibiotics mixed solution.

For example, the seed medium may comprise at least about 2500 U/mL IL-2, at least about 500 U/mL IL-6, at least about 1000 U/mL IL-12, at least about 10 µg/mL CD28 antibody or the antigen-binding fragment thereof, at least about 3 µg/mL OX-40 antibody or the antigen-binding fragment thereof, at least about 5 µg/mL CTLA-4 antibody or the antigen-binding fragment thereof, at least about 15 pg/mL TNFα, at least about 5 µM imatinib, at least about 5% human serum, the serum-free medium CTS^{™}OpTmizer^{™}, and the 100 U/mL PS dual-antibiotics mixed solution.

In the present application, the seed medium may comprise IL-2, IL-7, IL-15 and CD137 antibodies or antigen-binding fragments thereof, a CD28 antibody or an antigen-binding fragment thereof, a LAG-3 antibody or an antigen-binding fragment thereof, a PD-1 antibody or an antigen-binding fragment thereof, dasatinib, a human serum, a serum-free medium X-VIVO 15, and a PS dual-antibiotics mixed solution.

For example, the seed medium may comprise at least about 3000 U/mL IL-2, at least about 200 U/mL IL-7, at least about 300 U/mL IL-15, at least about 12 µg/mL CD137 antibody or the antigen-binding fragment thereof, at least about 5 µg/mL CD28 antibody or the antigen-binding fragment thereof, at least about 5 µg/mL LAG-3 antibody or the antigen-binding fragment thereof, at least about 3 µg/mL PD-1 antibody or the antigen-binding fragment thereof, at least about 3 µM dasatinib, at least about 5% human serum, the serum-free medium X-VIVO 15, and the 100 U/mL PS dual-antibiotics mixed solution.

In the present application, the seed medium may comprise IL-2, IL-6, IL-12, G-CSF, M-CSF, IFN-β, IFN-γ and CTLA-4 antibodies or antigen-binding fragments thereof, a PD-1 antibody or an antigen-binding fragment thereof, dasatinib, LYC-55716, GNE-1858, a human serum, a serum-free medium X-VIVO 15, and a PS dual-antibiotics mixed solution.

For example, the seed medium may comprise at least about 3000 U/mL IL-2, at least about 500 U/mL IL-6, at least about 500 U/mL IL-12, at least about 1000 U/mL G-CSF, at least about 500 U/mL M-CSF, at least about 500 U/mL IFN-β, at least about 800 U/mL IFN-γ, at least about 3 µg/mL CTLA-4 antibody or the antigen-binding fragment thereof, at least about 5 µg/mL PD-1 antibody or the antigen-binding fragment thereof, at least about 2.5 µM dasatinib, at least about 10 µM LYC-55716, at least about 4.5 nM GNE-1858, at least about 5% human serum, the serum-free medium X-VIVO 15, and the 100 U/mL PS dual-antibiotics mixed solution.

In the present application, the seed medium may comprise IL-2, IL-1α, IL-9, GM-CSF and CD137 antibodies or antigen-binding fragments thereof, a CD28 antibody or an antigen-binding fragment thereof, a LAG-3 antibody or an antigen-binding fragment thereof, a TIGIT antibody or an antigen-binding fragment thereof, CNI-1493, methylene blue, TWS119, a human serum, a serum-free medium AIM-V, and a PS dual-antibiotics mixed solution.

For example, the seed medium may comprise at least about 2500 U/mL IL-2, at least about 500 U/mL IL-1α, at least about 500 U/mL IL-9, at least about 800 U/mL GM-CSF, at least about 3 µg/mL CD137 antibody or the antigen-binding fragment thereof, at least about 3 µg/mL CD28 antibody or the antigen-binding fragment thereof, at least about 5 µg/mL LAG-3 antibody or the antigen-binding fragment thereof, at least about 2.5 µg/mL TIGIT antibody or the antigen-binding fragment thereof, at least about 2.5 µM CNI-1493, at least about 1 µM methylene blue, at least about 5 µM TWS119, at least about 5% human serum, the serum-free medium AIM-V, and the 100 U/mLPS dual-antibiotics mixed solution.

In the present application, the seed medium may comprise IL-2, IL-1α, IL-12, IL-21, G-CSF, M-CSF, IFN-α, IFN-β, IFN-γ and CD28 antibodies or antigen-binding fragments thereof, a CD40 antibody or an antigen-binding fragment thereof, a LAG-3 antibody or an antigen-binding fragment thereof, a PD-1 antibody or an antigen-binding fragment thereof, CNI-1493, dasatinib, GNE-1858, methylene blue, a human serum, a serum-free medium AIM-V, and a PS dual-antibiotics mixed solution.

For example, the seed medium may comprise about 3000 U/mL IL-2, about 200 U/mL IL-1α, about 500 U/mL IL-12, about 500 U/mL IL-21, about 300 U/mL G-CSF, about 800 U/mL M-CSF, about 500 U/mL IFN-α, about 300 U/mL IFN-β, about 300 U/mL IFN-γ, about 3 µg/mL CD28 antibody or the antigen-binding fragment thereof, about 5 µg/mL CD40 antibody or the antigen-binding fragment thereof, about 5 µg/mL LAG-3 antibody or the antigen-binding fragment thereof, about 3 µg/mL PD-1 antibody or the antigen-binding fragment thereof, about 3 µM CNI-1493, about 2 µM Dasatinib, about 5 nM GNE-1858, about 1.5 µM methylene blue, about 5% human serum, the serum-free medium AIM-V, and the 100 U/mLPS dual-antibiotics mixed solution.

In another aspect, the present application provides a seed cell of a tumor-infiltrating lymphocyte obtained by culturing using the seed cell medium of the present application, or a cell population thereof.

In another aspect, the present application provides a pharmaceutical composition, comprising the seed cell of the tumor-infiltrating lymphocyte or the cell population thereof according to the present application, and a pharmaceutically acceptable carrier.

In another aspect, the present application provides use of the seed cell of the tumor-infiltrating lymphocyte or the cell population thereof according to the present application, and the pharmaceutical composition according to the present application in the preparation of a medicament for treating a cancer.

The seed medium of the present application can be adapted to the expansion of tumor-infiltrating lymphocytes derived from different types of cancers. The seed medium of the present application shows a broad spectrum and universal applicability to cancer types. In some cases, the seed medium can be adapted to the culture of tumor infiltrating lymphocytes derived from different types of cancers, and may be adapted to the acquisition of seed cells of tumor infiltrating lymphocytes derived from different types of cancers, and a cell population thereof. In some cases, the seed medium of the present application and/or the pharmaceutical composition of the present application can be used to prepare a medicament for treating a cancer. In the present application, the cancer may comprise: cervical cancer (for example, which may comprise recurrent cervical cancer), ovarian cancer, poorly differentiated pelvic adenocarcinoma, intestinal cancer (for example, which may comprise intestinal cancer that has metastasized, such as liver metastases), gastric cancer, melanoma, breast cancer, and/or endometrial stromal sarcoma. For example, the cancer may be selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated pelvic adenocarcinoma, and bile duct cancer.

In another aspect, the present application provides use of the seed cell medium in the expansion of tumor-infiltrating lymphocytes.

In another aspect, the present application provides a method for culturing a seed cell for tumor-infiltrating lymphocytes, comprising the steps of: culturing isolated tumor-infiltrating lymphocytes in the seed cell medium of the present application.

In another aspect, the present application provides a method for expanding tumor-infiltrating lymphocytes, comprising the steps of: culturing isolated tumor-infiltrating lymphocytes in the seed cell medium of the present application to obtain seed cells of the tumor-infiltrating lymphocytes.

For example, the isolated tumor-infiltrating lymphocytes may be derived from a sample selected from the group consisting of: ascites from a subject in need, a primary tumor sample after surgical resection, a metastasis sample after synchronous and metachronous surgical resection, a needle biopsy sample, and a body fluid.

For example, the body fluid may comprise blood, a tissue fluid, a lymph fluid and/or a body cavity effusion. For example, the body fluid may comprise a peritoneal effusion (for example, which may be the peritoneal effusion of a patient with gastric cancer, or the peritoneal effusion of a patient with breast cancer) and/or a pleural fluid (for example, which may be the pleural effusion of a patient with endometrial stromal sarcoma).

For example, the isolated tumor-infiltrating lymphocytes may be derived from a tumor selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated pelvic adenocarcinoma, and bile duct cancer. For example, the isolated tumor-infiltrating lymphocytes may be derived from a tumor tissue of a cancer selected from the group consisting of: cervical cancer (for example, which may comprise a recurrent cervical cancer), ovarian cancer, poorly differentiated pelvic adenocarcinoma, intestinal cancer (for example, which may comprise a recurrent intestinal cancer with metastases, for example, liver metastases), gastric cancer, melanoma, breast cancer, and/or endometrial stromal sarcoma.

For example, the isolated tumor-infiltrating lymphocytes may be derived from tissue fragments obtained by cutting a tumor tissue resection, and the tissue fragments obatained by cutting the tumor tissue resection have a diameter of about 1 mm to about 10 mm, for example, about 2 mm to about 10 mm, about 3 mm to about 10 mm, about 4 mm to about 10 mm, about 5 mm to about 10 mm, about 6 mm to about 10 mm, about 7 mm to about 10 mm, or about 8 mm to about 10 mm.

For example, the temperature for the culture may be about 3-20 days. For example, the time for the culture may be about 3-15 days. For example, the time may be about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, or about 20 days.

For example, the temperature for the culture may be about 30-42°C. For example, the temperature may be at least about 30°C, at least about 31°C, at least about 32°C, at least about 33°C, at least about 34°C, at least about 35°C, at least about 36°C, at least about 37°C, at least about 38°C, at least about 39°C, at least about 40°C, at least about 40°C, or at least about 42°C.

For example, the CO₂ concentration for the culture is about 1%-10%. For example, the CO₂ concentration may be at least about 1%, at least about 2%, at least about 3%, at least about 4%, at least about 5%, at least about 6%, At least about 7%, at least about 8%, at least about 9%, or at least about 10%.

In the present application, the use of the seed medium of the present application can significantly increase the cellular activity of the seed cells of tumor-infiltrating lymphocytes (for example, it can significantly increase the total counts of the seed cells of tumor-infiltrating lymphocytes, for example, by enabling the number of the seed cells of tumor-infiltrating lymphocytes to reach the order of magnitude of at least about 10⁸; for example, it can significantly increase the celllular viability of the seed cells of tumor-infiltrating lymphocytes, for example, by enabling the cell viability of the seed cells of tumor-infiltrating lymphocytes to reach at least about 85%; and for example, it can significantly increase the amount of cytokines (such as IFN-γ) secreted by the seed cells of tumor-infiltrating lymphocytes). In the present application, the use of the seed medium of the present application can significantly increase the proportion of CD3⁺ cells in the seed cells of tumor-infiltrating lymphocytes.

In the present application, the use of the seed medium of the present application can greatly improve the frequency of a portion of TCR clones, in the peripheral blood of a donor subject, of an infused product TIL of the seed cells of the tumor-infiltrating lymphocytes after infusion into the donor subject (for example, a tumor patient); and the similarity between the TCR repertoire of the peripheral blood of the donor subject and the TCR repertoire of the TIL infusion product is also significantly increased, and can be maintained for a long time. Furthermore, after the donor subject undergoes the autologous infusion of TILs cultured in the seed medium of the present application, the lymphocytes in the donor subject can robustly proliferate.

Not wishing to be bound by any particular theory, the following examples are merely to illustrate the fusion protein, preparation methods and uses and the like according to the present application, and are not intended to limit the scope of the present invention.

### Examples

### Example 1 TIL seed cell medium

The TIL seed cell medium was prepared using the following components (Catalog No.).

| | | | |
|---|---|---|---|
| IL-1α | R&D 200-LA-010 | OX-40 mAb | R&D MAB10543-100 |
| IL-1β | R&D 201-LB-025 | LAG-3 mAb | R&D MAB23193 |
| IL-2 | R&D 201-GMP-01M | TIGIT mAb | R&D MAB7898 |
| IL-4 | R&D 204-GMP-01M | CTLA-4 mAb | R&D MAB325-500 |
| IL-6 | R&D 206-GMP-01M | PD-1 mAb | R&D MAB10861 |
| IL-7 | R&D 207-GMP-01M | TNF-α | R&D 210-GMP-100 |
| IL-9 | R&D 209-ILB-050/CF | RRx-001 | MCE HY-16438 |
| IL-10 | R&D 217-IL-025/CF | Sunitinib | MCE HY-10255A |
| IL-12 | R&D 219-GMP-01M | CNI-1493 | MCE HY-15509A |
| IL-15 | R&D 247-GMP-01M | Imatinib | MCE HY-15463 |
| IL-18 | Biotechne 9124-IL-500 | CAL-101 | MCE HY-13026 |
| IL-21 | R&D 8879-GMP-01M | Dasatinib | MCE HY-10181 |
| G-CSF | R&D 214-CS-025 | LYC-55716 | MCE HY-104037 |
| GM-CSF | R&D 215-GMP-01M | GNE-1858 | MCE HY-135892 |
| M-CSF | R&D 216-GMP-500 | Methylene blue | MCE HY-14536 |
| IFN-α | R&D 11100-1 | TWS119 | MCE HY-10590 |
| IFN-β | R&D 8499-IF-010 | Human AB serum (v/v%) | Sigmaaldrich H4522 |
| IFN-γ | R&D 285-GMP-01M | 100×PS dual-antibiotics | Thermo Fisher15140122 |
| CD137 mAb | R&D AF838 | AIM-V | Thermo Fisher 0870112BK |
| CD28 mAb | R&D MAB342-500 | X-VIVO 15 | Lonza BE02-060F |
| CD40 mAb | R&D MAB6321-500 | CTS^{™} OpTmizer^{™} | Thermo FisherA1048501 |

Different TIL seed cell media were formulated according to Table 1 to Table 3. After the addition of a serum and dual antibiotics to a basal medium proportionally, the basal medium could be stored at 4°C for no more than 1 month. Before use, the medium at 4°C was taken out and preheated in a water bath at 37°C, and then other components (various interleukins, colony stimulating factors, interferons, TNF-α, various antibodies and other molecules) were added to a working concentration for TIL culture. In addition, as a contrast, a CM1 medium (hereinafter referred to as CM1) containing 6000 IU/mL IL-2, Glutamax and antibiotics was prepared according to the formula and method described in Example 5 in the specification of CN110099998A, and was for use in the pre-REP culture of TILs (corresponding to the seed cells herein) derived from tumor samples.

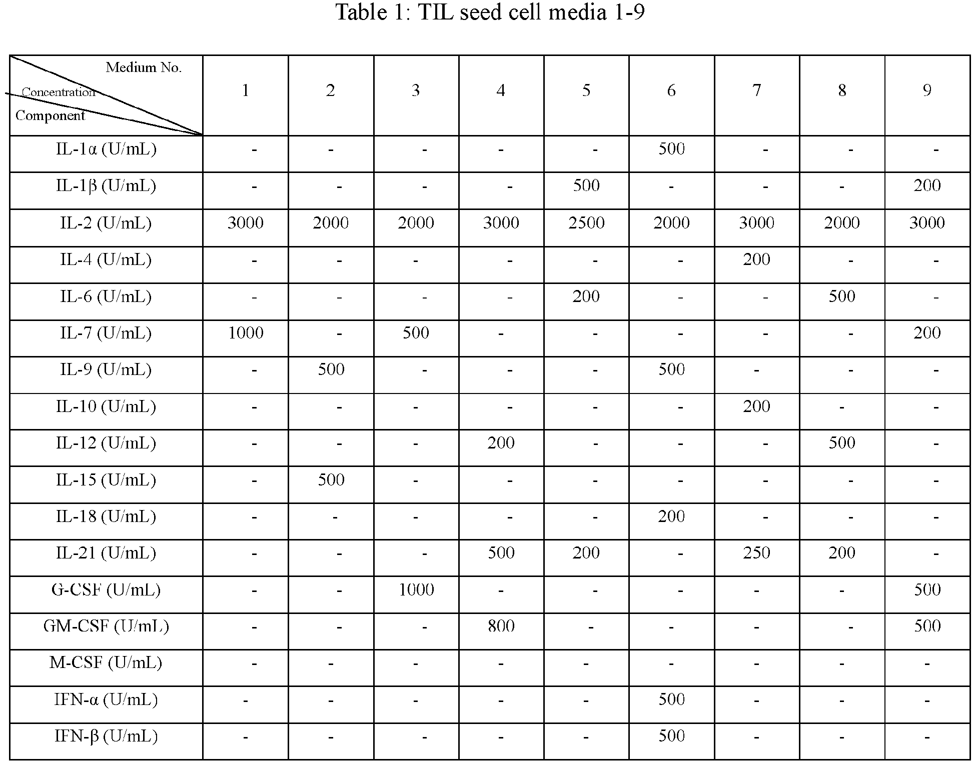

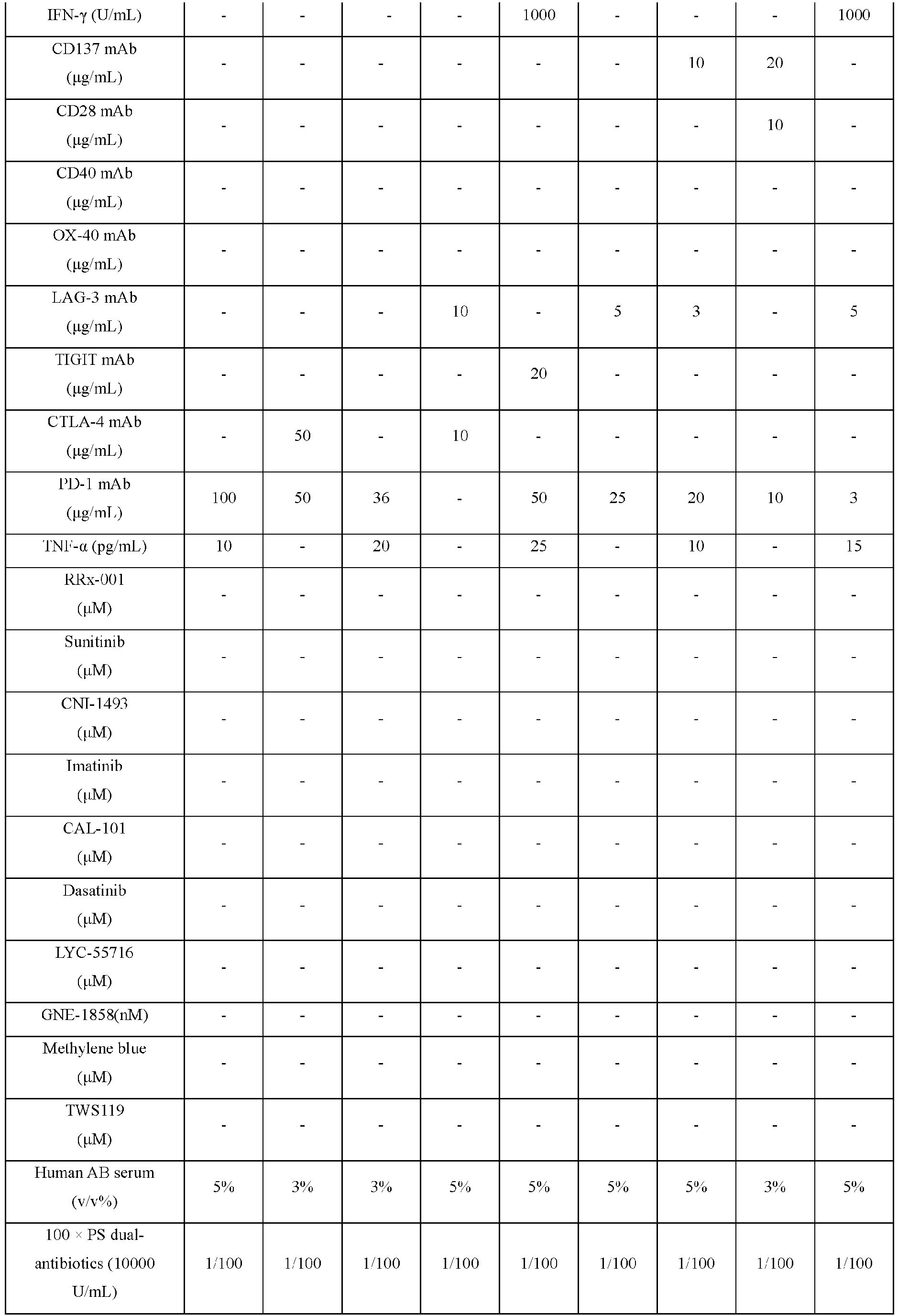

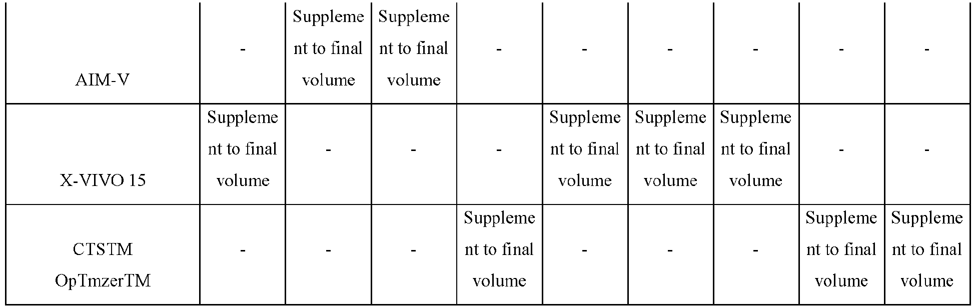

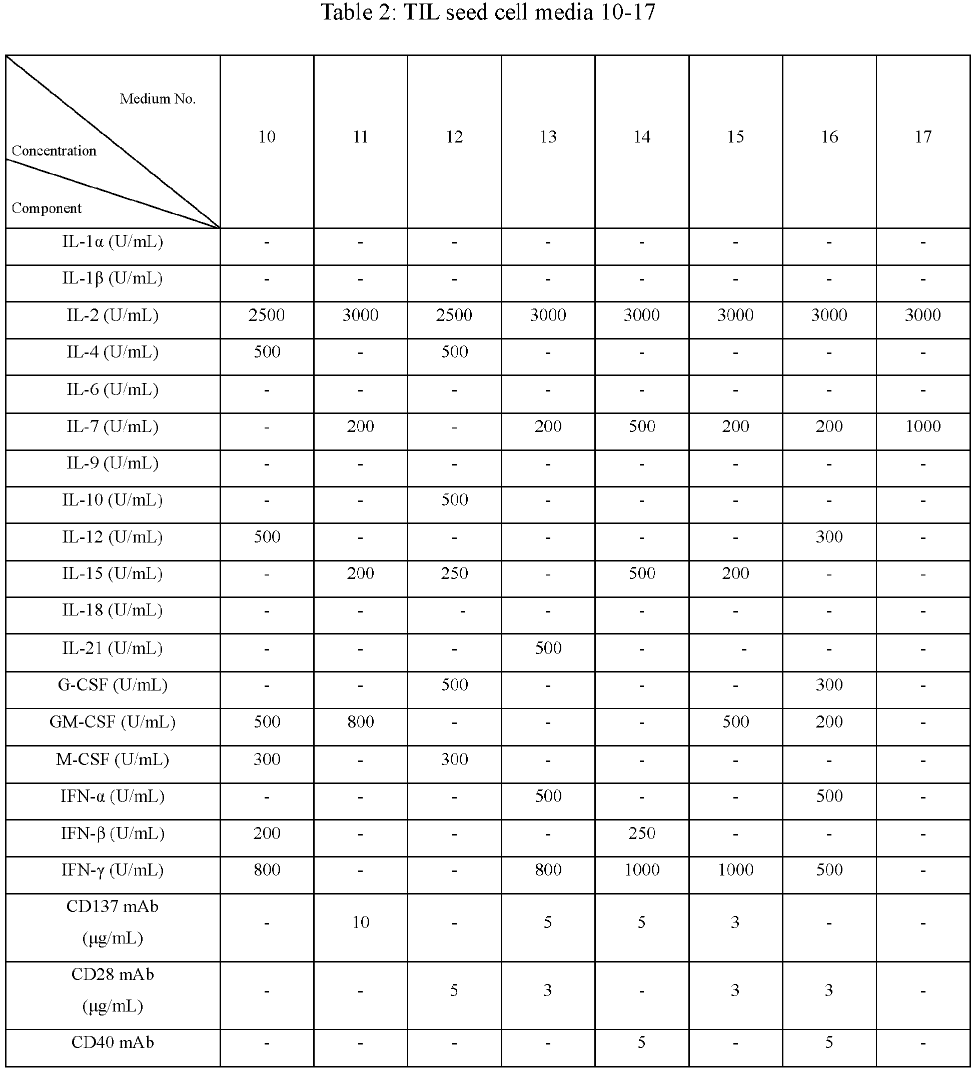

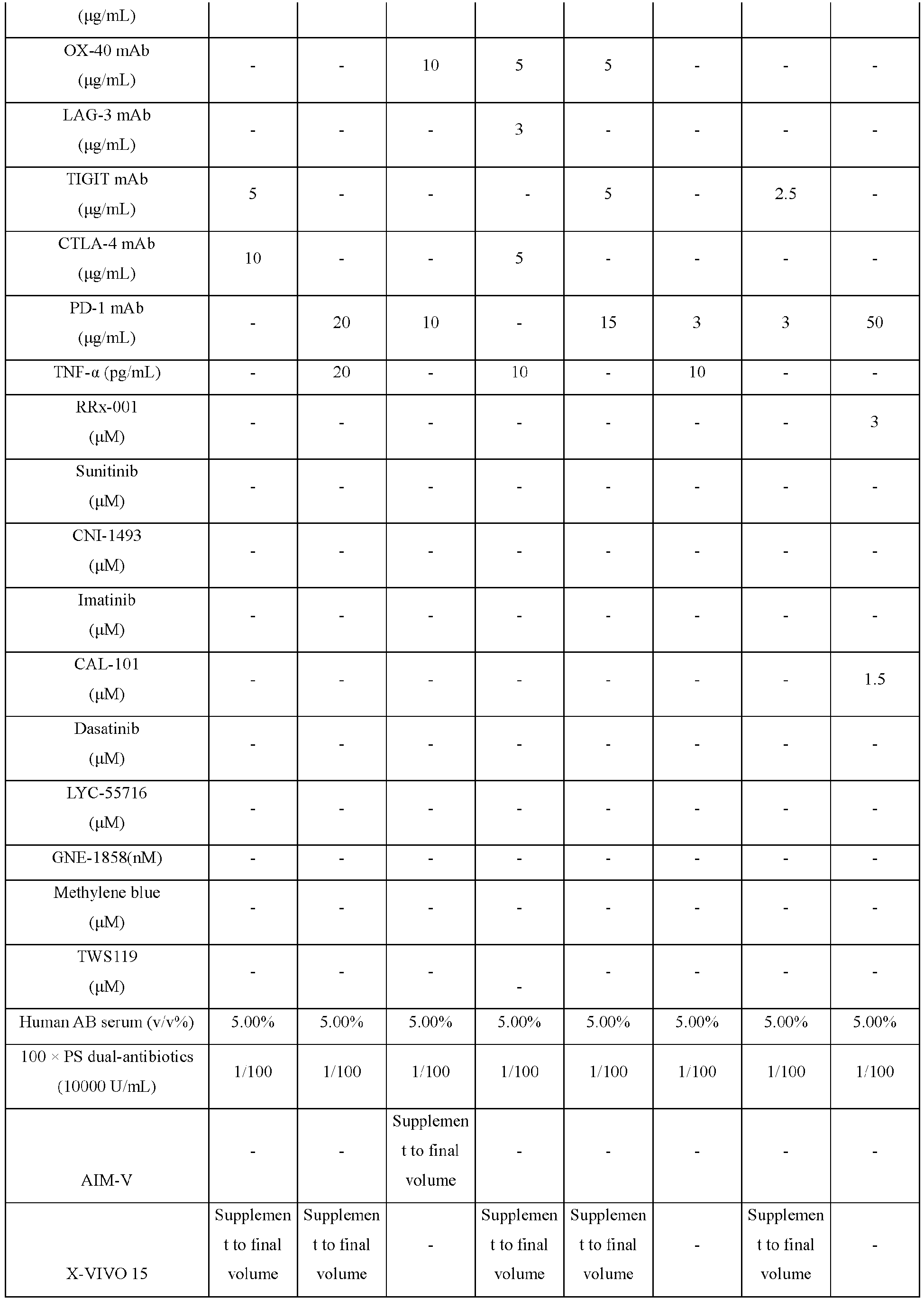

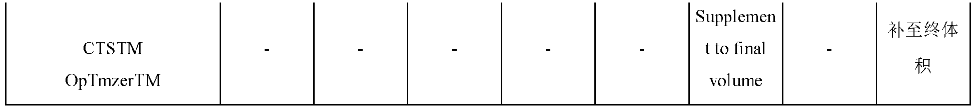

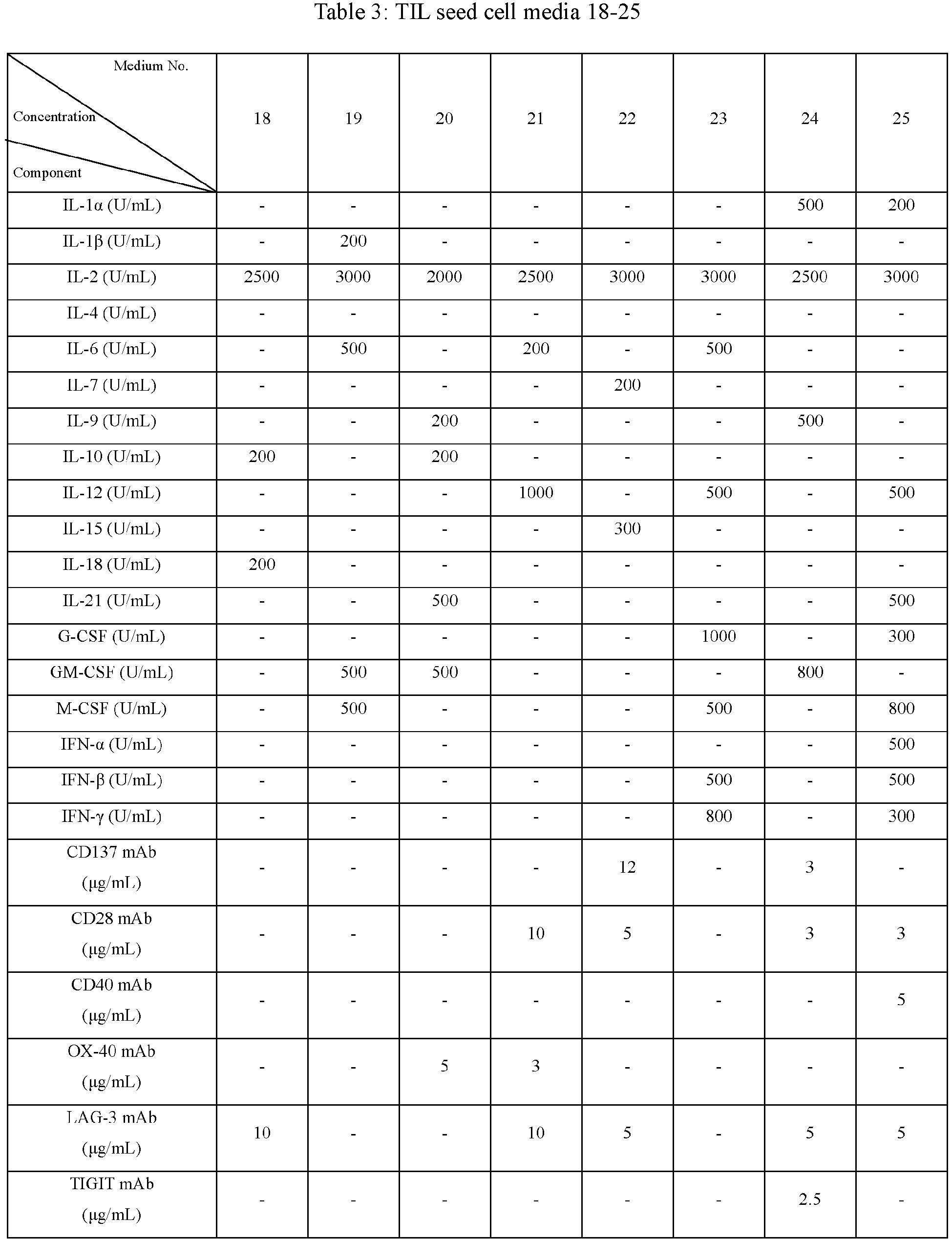

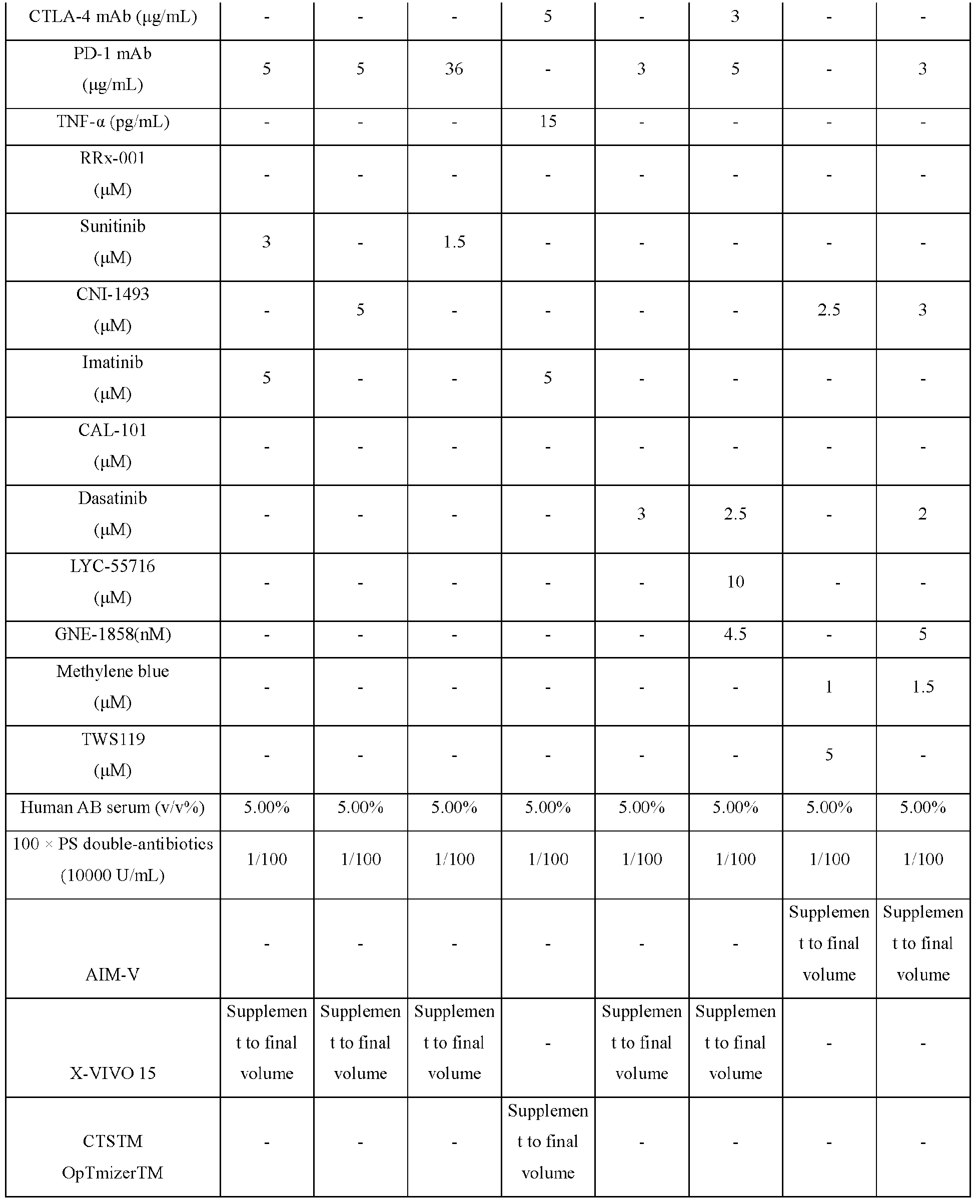

### Example 2 Treatment of solid tumor samples (surgically resected tissues) and TIL seed cell culture

1) Normal saline containing penicillin at a final concentration of 100 U/mL, streptomycin at a final concentration of 100 µg/mL and gentamicin at a final concentration of 50 µg/mL was prepared for later use.
2) In a BSL-2 cabinet, the freshly isolated tumor tissue samples were washed in a sterile environment in a 10 cm petri dish into which 30 mL of the normal saline prepared in step 1) was added, and then transferred to a 10 cm new dish into which 30 mL of the normal saline prepared in step 1) was added, and the washing was conducted three times.
3) Adipose tissues and necrotic tissues were removed using a sterile surgical blade; the tumor tissues were cut into small pieces with the dimension of 3×3×3 mm³; and 12 pieces of randomly selected tumor tissues were placed in each G-REX10 culture tank (purchased from Wilsonwolf), with each G-REX10 culture tank corresponding to one type of TIL seed medium prepared in Example 1. The excess pieces of tumor tissues were cryopreserved in liquid nitrogen using a CryoStor10 (purchased from BioLifeSolutions) cryoprotectant by means of a controlled-rate freezer.
4) Each group of TIL seed cell medium prepared in Example 1 was added to a different G-REX10 culture tank, 40 mL per tank; and those pieces of tumor tissues were cultured at 37°C in the presence of 5% CO₂. On Day 12, the TIL seed cells were harvested and total cell counts and cellular viability were measured; the phenotype of the cells was detected using a flow cytometer; and the secretion level of IFN-γ was detected with an HTRF IFN-γ detection kit (Cisbio Human IFN gamma kit, Catalog No.: 62HIFNGPET) according to the method described in the user manual.

### Example 3 Treatment of in vivo effusion (ascite, pleural effusion) in tumor patients and TIL seed cell culture

The body effusions (ascites T012 and T013 and pleural effusion T014) derived from tumor patients were centrifuged at 300 g for 8 minutes. After removing the supernatant, the cell pellets were washed three times with D-PBS, during which the cell pellets were centrifuged at 300 g for 8 minutes each time. Finally, the cells were resuspended in the TIL seed medium and cultured at 37°C in the presence of 5% CO₂. On Day 12, the TIL seed cells were harvested and total cell counts and cellular viability were measured; the phenotype of the cells was detected using a flow cytometer; and the secretion level of IFN-γ was detected with the HTRF IFN-γ detection kit (Cisbio Human IFN gamma kit, Catalog No.: 62HIFNGPET) according to the method described in the user manual.

The TIL seed cells in the ascite or pleural effusion in each of the following groups were derived from 6 mL of body effusion.

The information of each tumor tissue (body effusion) treated in Example 3 was shown in Table 4:

**Table 4**

| **Sample No.** | **Tumor type** |
|---|---|
| T001 | Cervical cancer |
| T002 | Ovarian cancer |
| T003 | Poorly differentiated pelvic adenocarcinoma |
| T004 | Cervical cancer (recurrent) |
| T005 | Liver metastasis of intestinal cancer |
| T006 | Gastric cancer |
| T007 | Gastric cancer |
| T008 | Gastric cancer |
| T009 | Intestinal cancer (needle biopsy) |
| T010 | Cervical cancer (needle biopsy) |
| T011 | Melanoma (needle biopsy) |
| T012 | Gastric cancer (ascite) |
| T013 | Breast cancer (ascite) |
| T014 | Endometrial stromal sarcoma (pleural effusion) |
| T015 | Cervical cancer (recurrent) |
| T016 | Cervical cancer |

The tumor tissues and body effusions treated in Example 2 and Example 4 were cultured in the medium groups arranged in Table 5, in which "+" indicates that the numbered medium was used, and "-" indicates that the medium was not used. CM1 medium was provided for tumor tissues T003, T005 and T007 as a control.

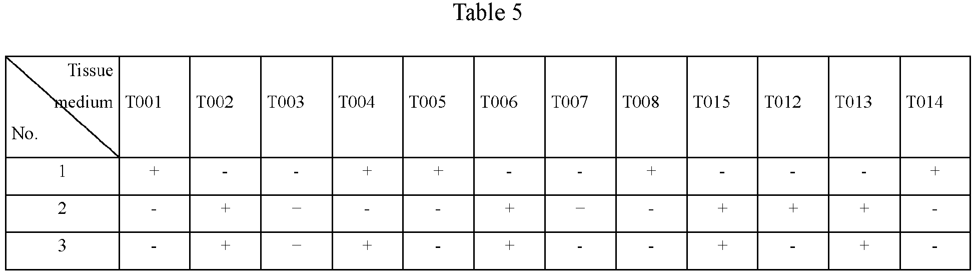

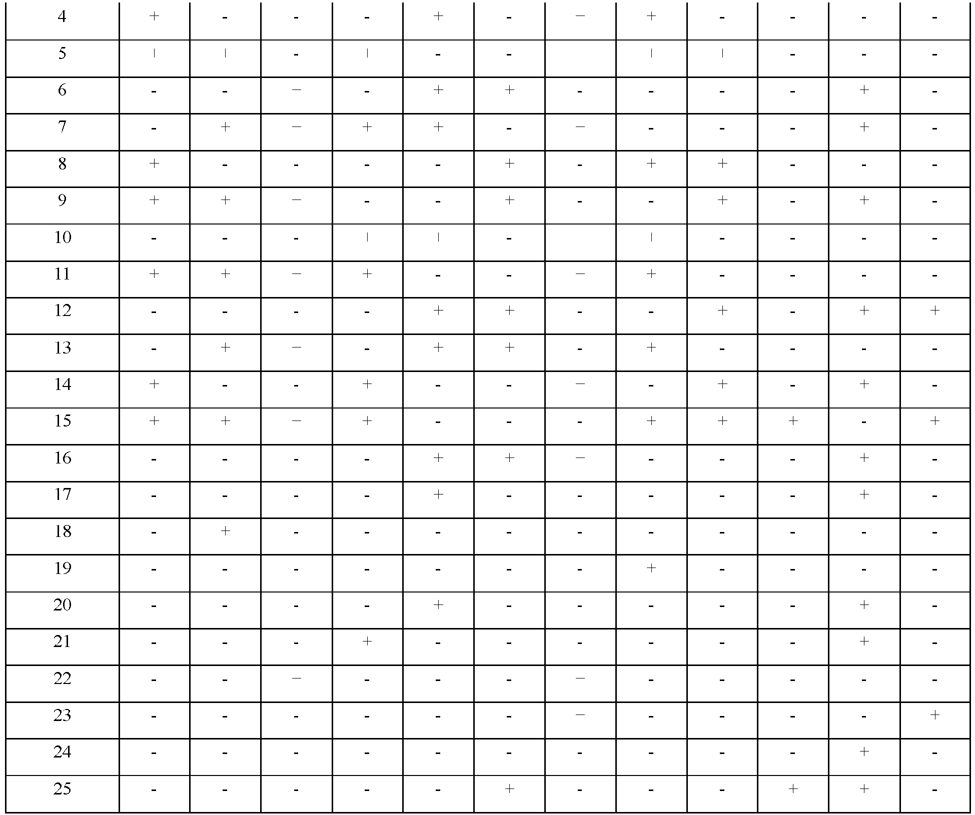

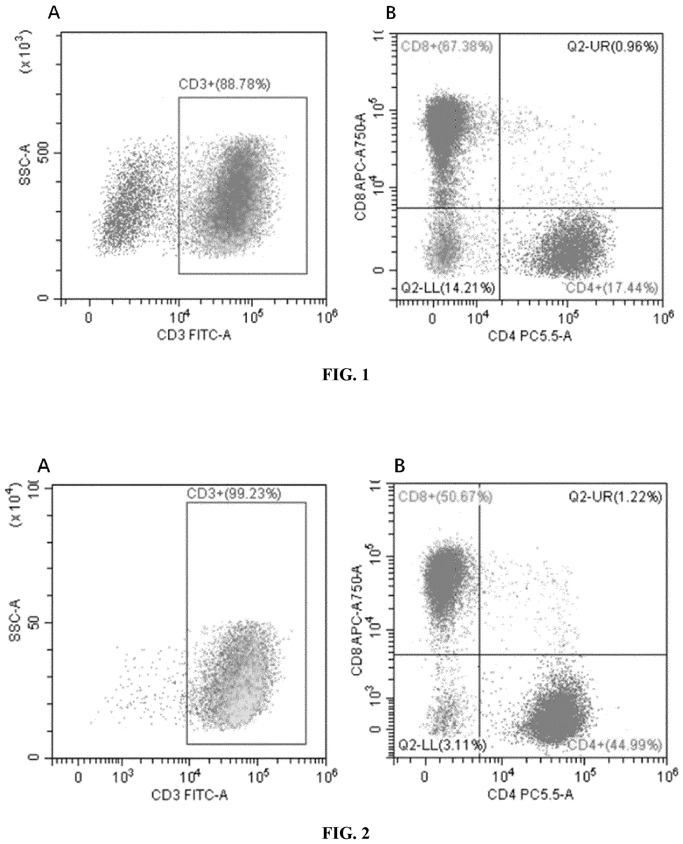

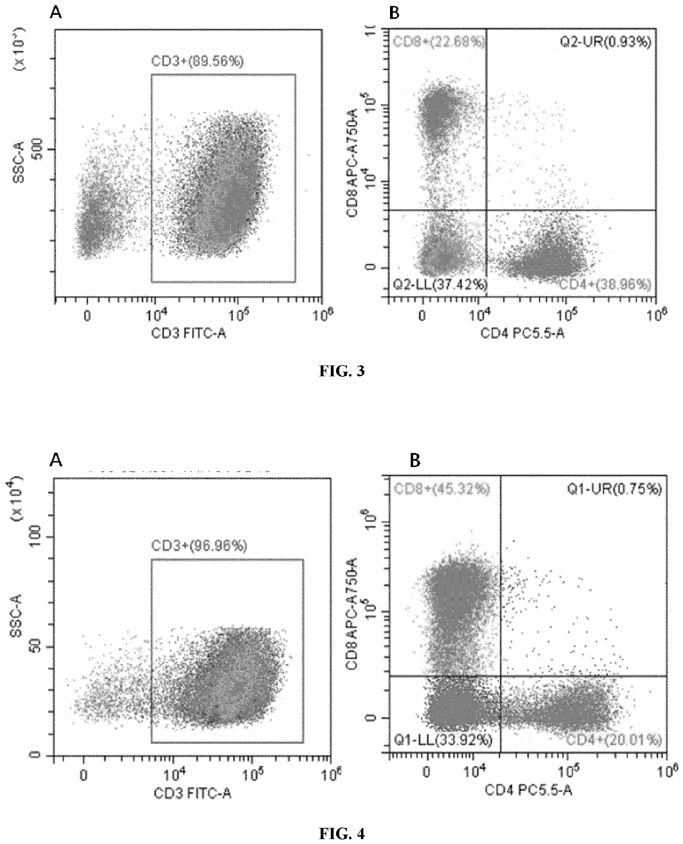

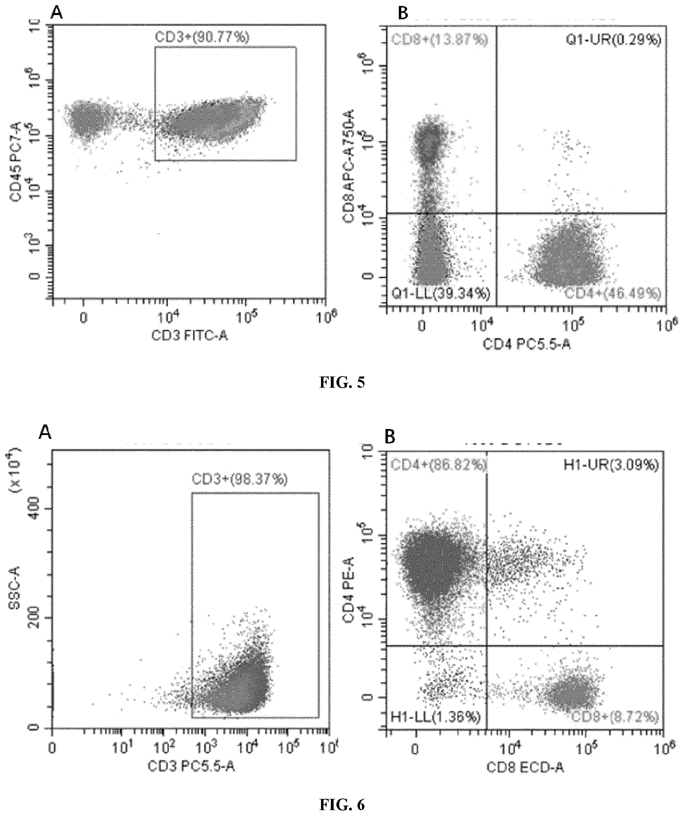

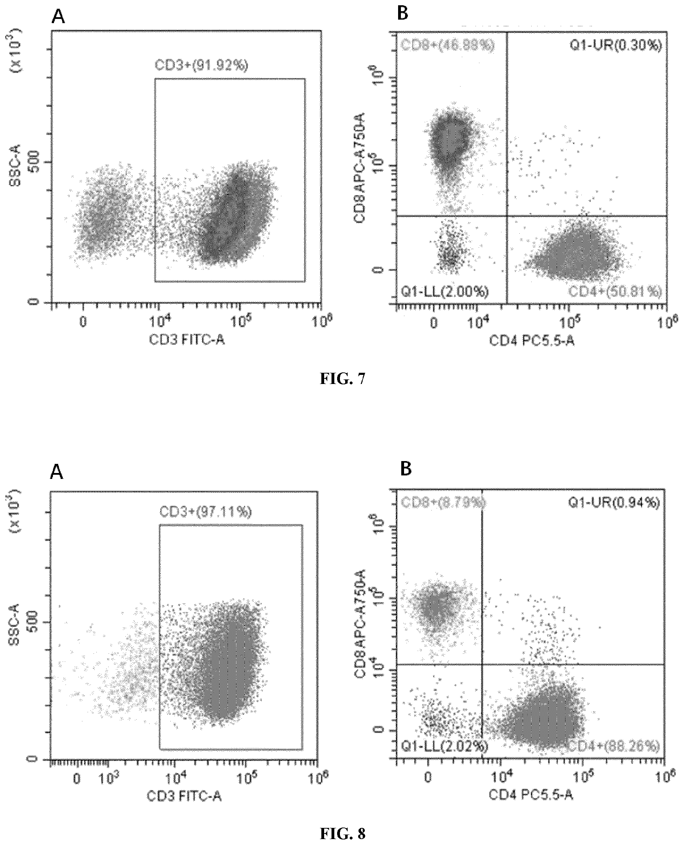

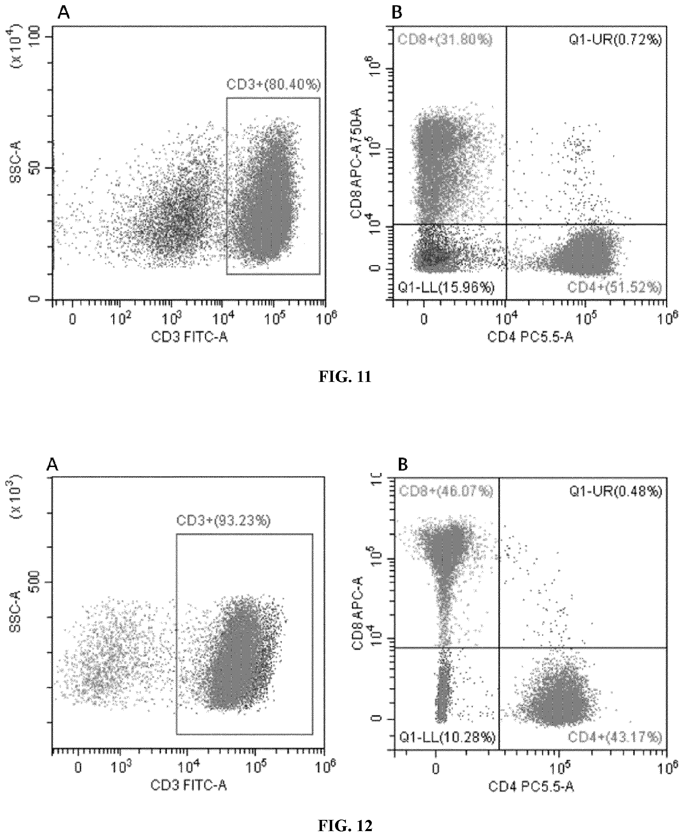

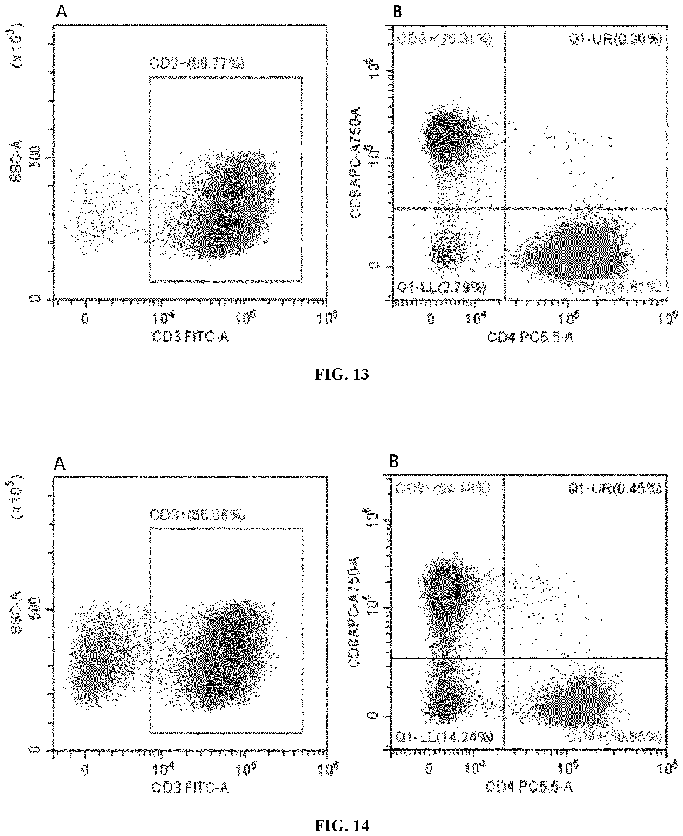

The culture results were shown in Table 6 to Table 17 and FIG. 1 to FIG. 15. Among them, Table 6 to Table 17 listed the TIL seed cell culture results of T001, T002, T003, T004, T005, T006, T007, T008, T015, T012, T013, and T014 in Table 5, respectively; FIG. 1 to FIG. 12 showed the representative flow cytometric analysis results of T001, T002, T003, T004, T005, T006, T007, T008, T015, T012, T013, and T014, respectively, after the TIL seed cell culture using the seed cell medium of the present application; and FIG. 13, FIG. 14, and FIG. 15 were the flow cytometric analysis results of T002, T003 and T007 cultured in the CM1 medium, respectively.

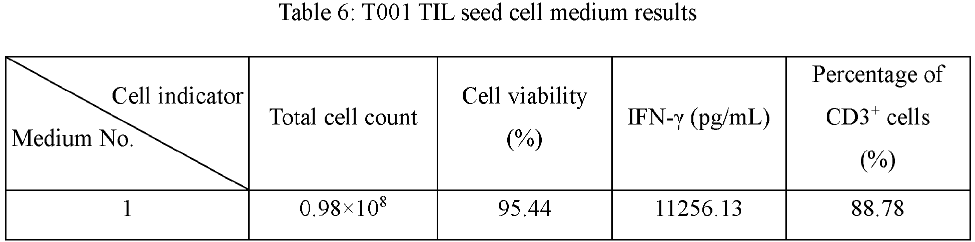

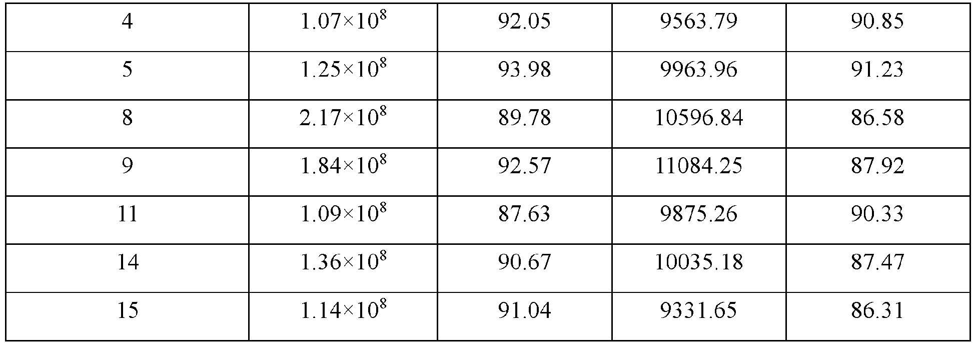

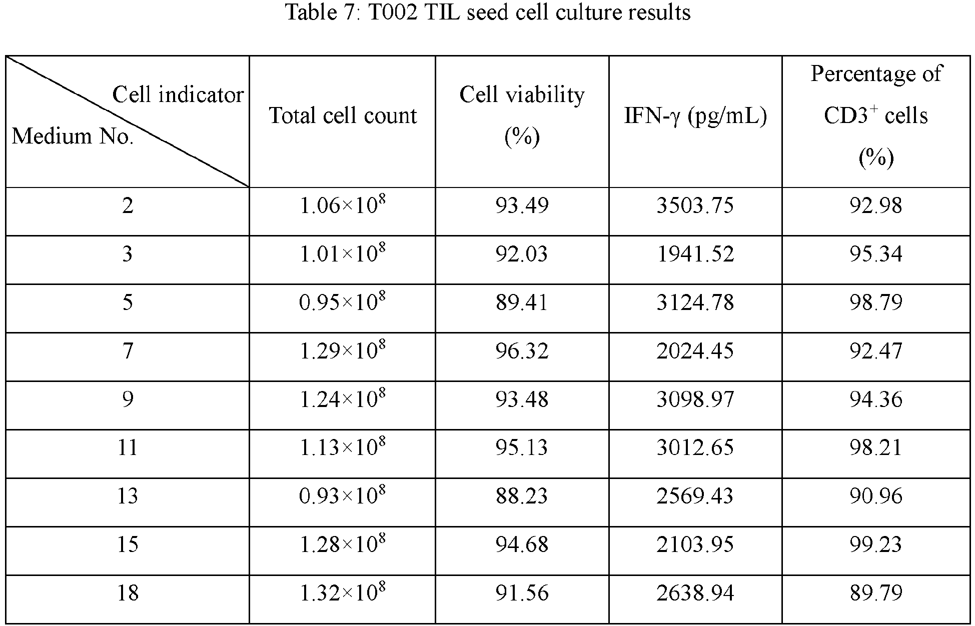

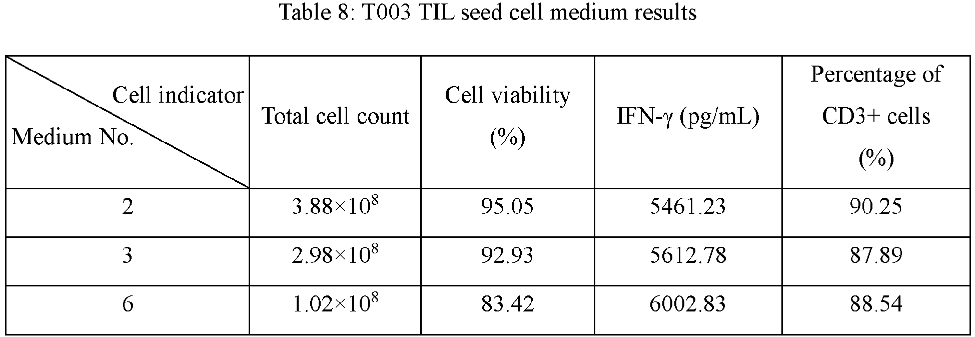

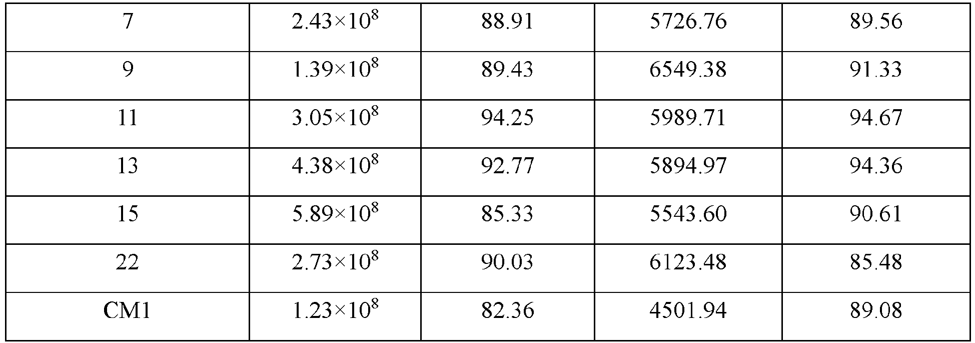

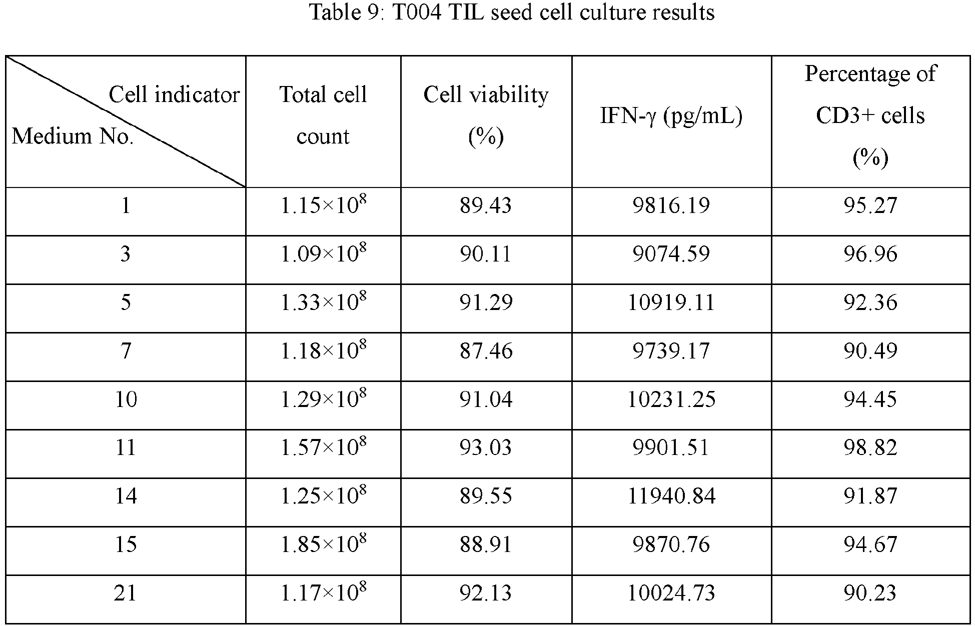

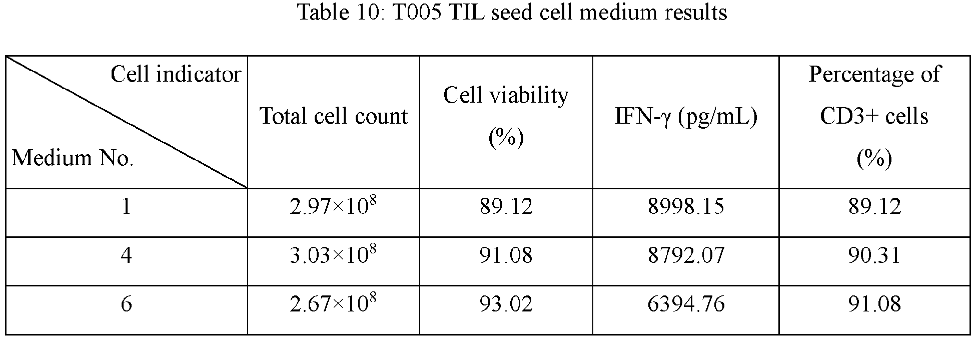

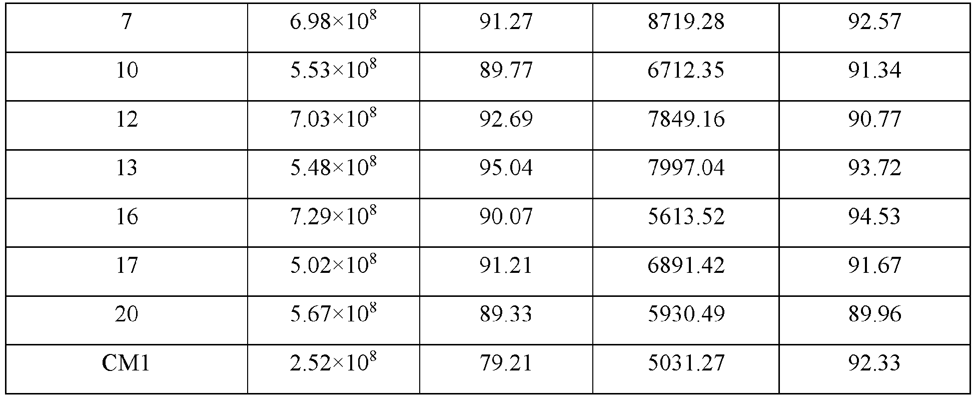

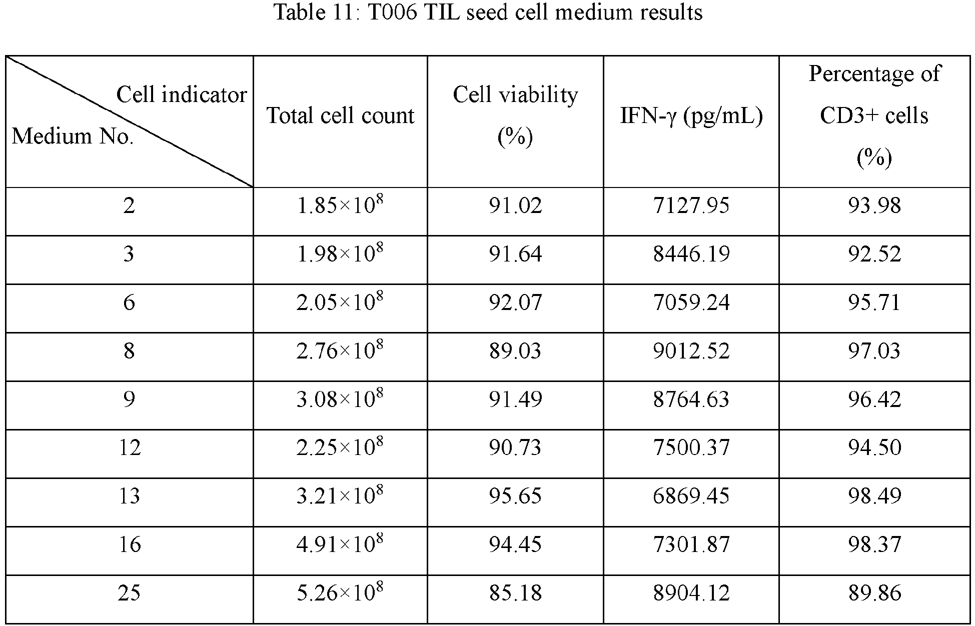

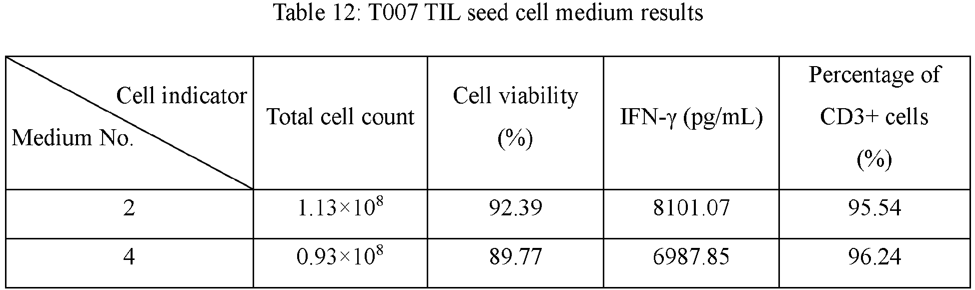

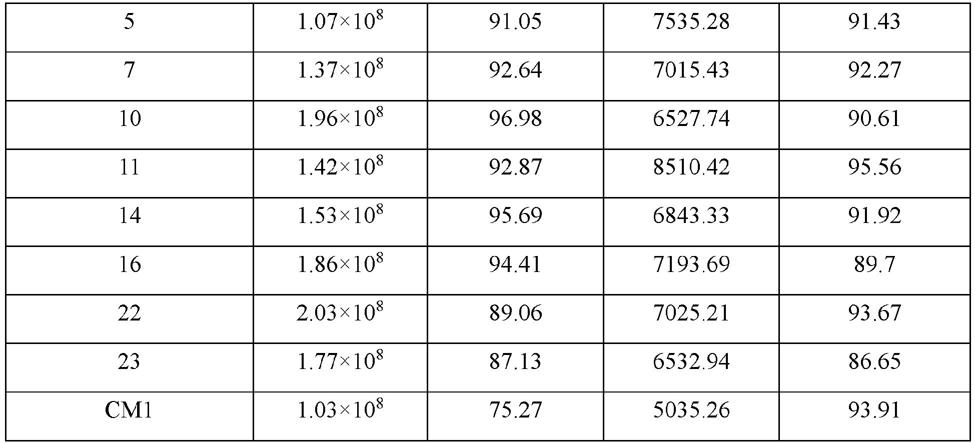

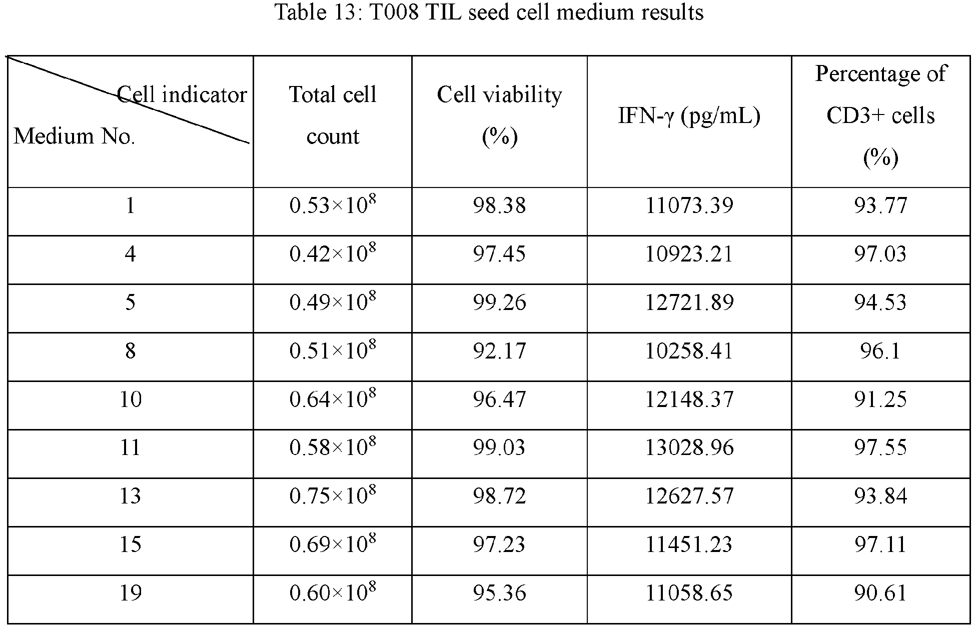

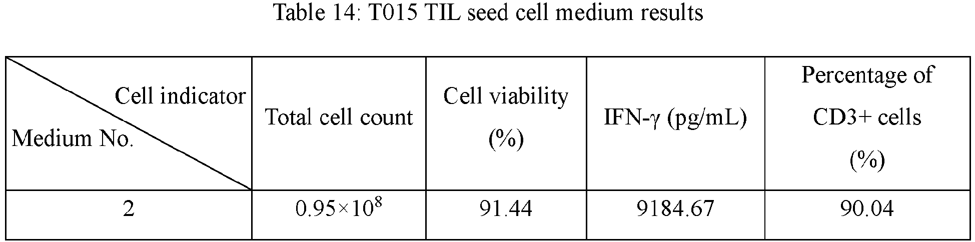

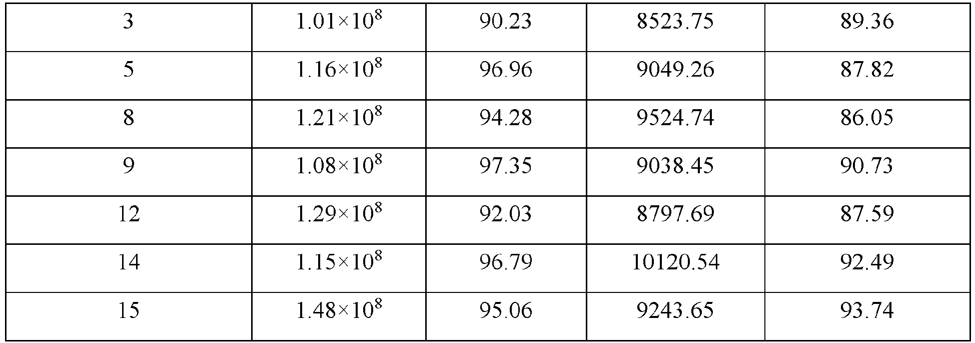

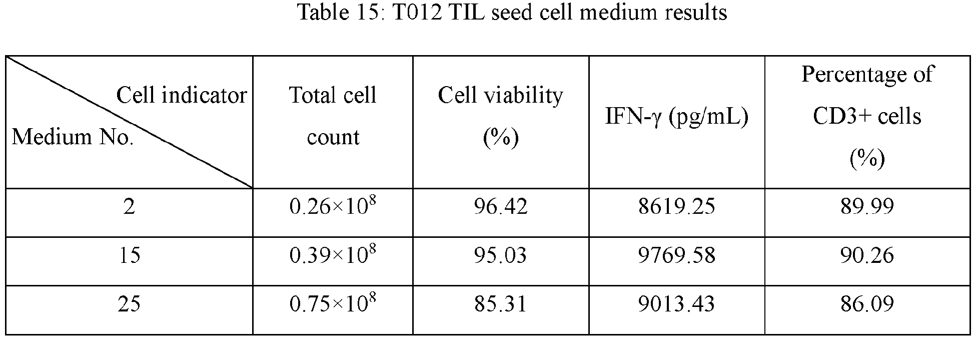

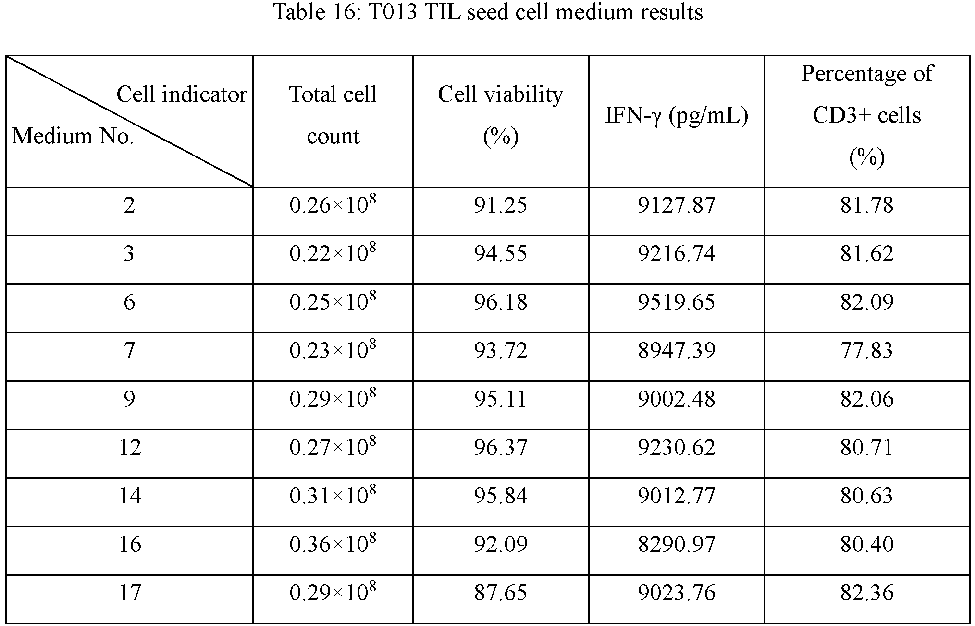

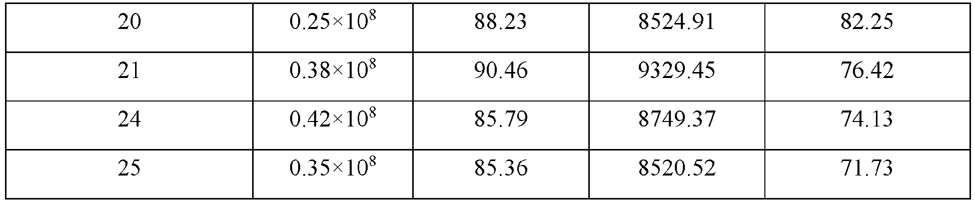

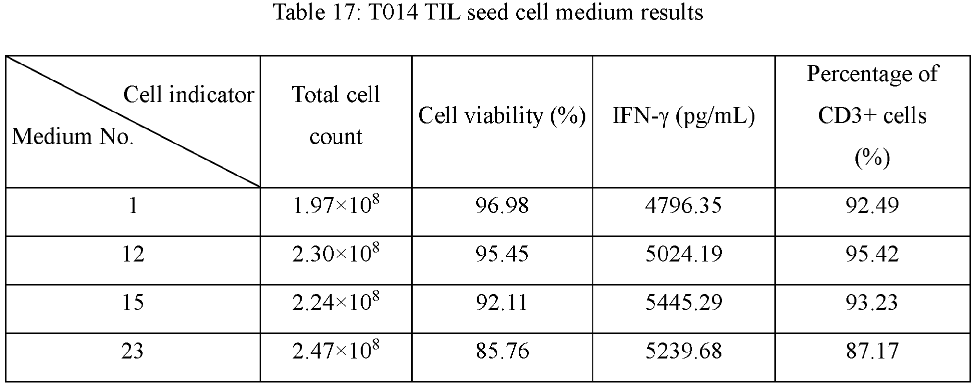

T016 was cultured with the medium No. 15, where the total count of cells was 1.13×10⁸, the cell viability was 95.01%, the concentration of secreted IFN-γin the supernatant was 8446.32 pg/mL, and the percentage of CD3⁺ cells was 92.64%.

Table 6 to Table 14 and the results of T016 showed that, on Day 12 when the solid tumor tissues cultured with the seed cell medium in each group in the present application were harvested, all the total counts of cells could reach at least about 10⁸ cells, and for some tumor samples, such as T003, T005 and T006, the total counts of cells could even reach up to about 6×10⁸ cells and exceed 7×10⁸ cells. Furthermore, although the total count of cells harvested from each of the three samples of T003, T005 and T007 that were cultured in parallel using CM1 also exceeded 1×10⁸ or 2×10⁸ cells, the total count of cells was relatively lower than that of cells cultured in the mediums of other groups corresponding thereto. The viability of the TIL seed cells cultured in the seed medium of each group of the present application was relatively high, which was above 85% and mostly exceeded 90%. However, the viability of the seed cells harvested from the three samples T003, T005 and T007 that were cultured in parallel with CM1 was significantly lower than the viability of the cells cultured in other medium groups corresponding thereto, where the viabilities of the CM1 seed cells of two samples T005 and T007 were both lower than 80%. In addition, the IFN-γ secretion of the TIL seed cells cultured using the seed medium of each group of the present application was at a relatively high level. The IFN-γ secretion of the TIL seed cells harvested from the three samples T003, T005 and T007 cultured in parallel using CM1 was also significantly lower than those corresponding thereto.

FIG. 1 to FIG. 9 showed that the flow cytometry phenotypes of TIL seed cells cultured from solid tumor tissues using the seed medium of each group in the present application were normal; the percentage of CD3⁺ cells in most of samples was more than 90%; and the percentage of CD3⁺ cells in some of the samples such as T002 and T006 was around 99%. The same results were also shown in the percentage data of CD3⁺ cells in Tables 6-14. The ratio of the percentage of CD4⁺ to the percentage of CD8⁺ in most of samples were close to 1:1, and the percentage of CD8⁺ cells in some of the samples such as T006 and T012 was high. FIG. 1A to FIG. 9A showed the percentages of CD3⁺ cells, respectively, and FIG. 1B to FIG. 9B showed the percentages of CD4⁺ cells, respectively. FIG. 1 to FIG. 9 sequentially showed the cell phenotype flow charts of T001, T002, T003, T004, T005, T006, T007, T008 and T015, which were cultured using the medium No. 1, the medium No. 3, the medium No. 2, the medium No. 10, the medium No. 7, the medium No. 8, the medium No. 16, the medium No. 11, and the medium No. 15, respectively.

The results in Table 15 to Table 17 showed that the TIL seed cells could also be successfully cultured from body effusions (such as pleural effusions and ascites) of tumor patients by using the seed cell medium of each group of the present application. For some of the samples, more than 2×10⁸ TIL seed cells could even be cultured from the body effusion as little as 10 mL or less. Moreover, the viability of TIL seed cells cultured from the body effusion by using the seed medium in each group was high, all higher than 85% and mostly higher than 90%; and meanwhile, the IFN-γ secretion of TIL seed cells in all groups was also at a high level.

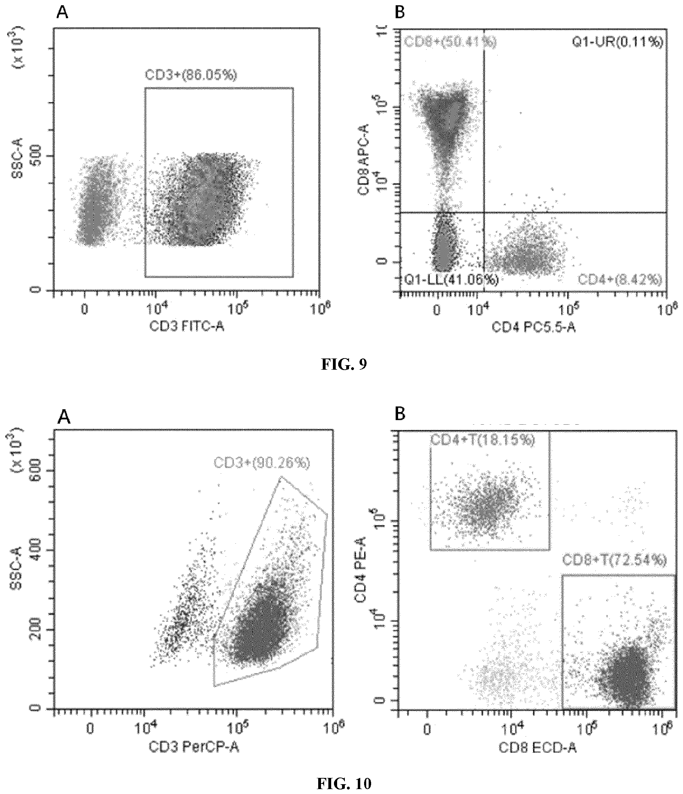

FIG. 10 to FIG. 12 showed that the flow cytometry phenotypes of TIL seed cells cultured from body effusion of tumor patients by using each seed medium group of the present application were normal; the percentages of CD3⁺ cells in all cases were more than 80%; and the percentages of CD3⁺ cells in the seed cells of T012 and T014 were more than 90%. The same results were also shown in the percentage data of CD3⁺ cells in Tables 15-17. FIG. 10 to FIG. 12 sequentially showed the cell flow cytometry phenotype charts of T012, T013, and T014, which were cultured using the medium No. 25, the medium No. 9, and the medium No. 12, respectively.

FIG. 13 to FIG. 15 sequentially showed that the flow cytometry phenotypes of TIL seed cells obtained by culturing three solid tumor tissue samples of T003, T005 and T007 in the CM1 medium were normal, with high percentage of CD3⁺ cells, where the percentages of CD3⁺ cells of T003 and T007 were around 99%, and the percentages of CD4⁺ cells of the two were significantly higher than those of the TIL seed cells cultured and harvested in other medium groups corresponding thereto.

### Example 4 Treatment of solid tumor samples (tissues obtained by needle biopsy) and TIL seed cell culture

1) Normal saline containing penicillin at a final concentration of 100 U/mL, streptomycin at a final concentration of 100 µg/mL, and gentamicin at a final concentration of 50 µg/mL was prepared for later use.
2) In a BSL-2 cabinet, the tumor tissues obtained by biopsy using a fine needle (with a diameter of 1 mm) were washed in a sterile environment in a 10 cm petri dish into which 30 mL of the normal saline prepared in step 1) was added, and then transferred to a 10 cm new dish into which 30 mL of the normal saline prepared in step 1) was added, and the washing was conducted three times.
3) The tumor tissues obtained by needle biopsy were cut using a sterile surgical blade into small pieces with a diameter of 1×1×3mm³; three pieces of randomly selected tumor tissues were placed in a well of each G-REX24 culture plate (purchased from Wilsonwolf), with each well of each G-REX24 culture plate corresponding to a TIL seed medium of a specific formula of Example 1 of the present application; CM1 medium was used as a control; and 40 mL of medium was added to each well.
4) The tumor tissues obtained by needle biopsy were cultured at 37°C in the presence of 5% CO₂. On Day 12, the TIL seed cells were harvested and total cell counts and cellular viability were measured; the phenotype of the cells was detected using a flow cytometer; and the secretion level of IFN-γ was detected with an HTRF IFN-γ detection kit (Cisbio Human IFN gamma kit, Catalog No.: 62HIFNGPET) according to the method described in the user manual.

The groups and culture results of the tumor tissue samples obtained by needle biopsy were shown in Table 18 to Table 20.

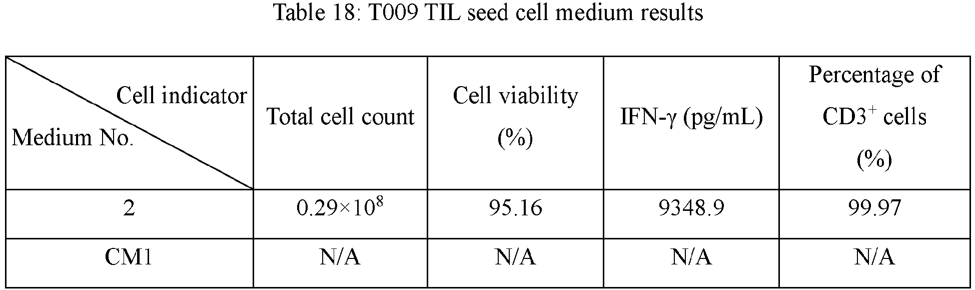

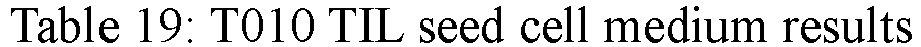

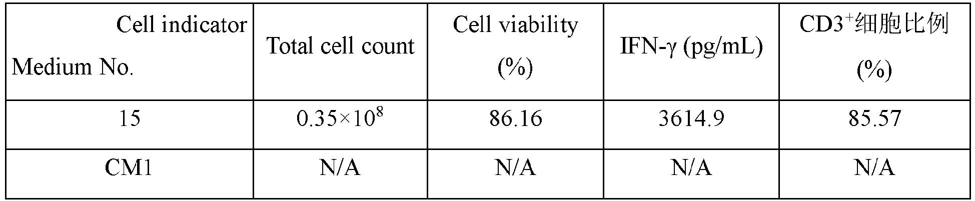

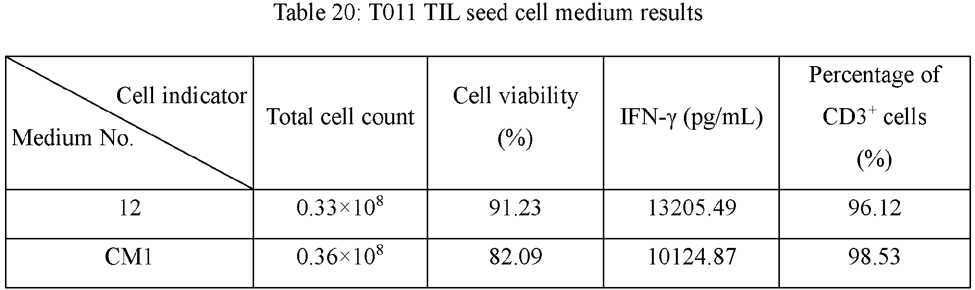

The results in Table 18 to Table 20 showed that, after 12 days of culture using the TIL seed cells No. 2, No. 15 and No. 12 enumerated in Example 1, respectively, around 0.3×10⁸ TIL seed cells could be obtained for the three needle biopsy tumor tissue samples T009, T010, and T011. After the aforementioned tissues were cultured in parallel using the CM1 medium, a roughly equivalent number of TILs could be obtained only from the malignant melanoma tissue sample T011 obtained by needle biopsy, and the other two tissue samples obtained by needle biopsy showed no obvious TILs under microscopic observation after 12 days. Compared with CM1 TIL, the TIL seed cells derived from T011 tumor tissues obtained by needle biopsy and cultured in the medium No. 12 showed higher viability and IFN-γ secretion, and the percentages of CD3⁺ cells was roughly the same in the two.

In addition, the cell viability of TILs derived from the above three tumor tissues obtained by needle biopsy and cultured using the TIL seed cell media No. 2, No. 15 and No. 12 was also greater than 86%, and up to 95% or more, and the level of IFN-γ secretion and the percentage of CD3⁺ cells was also at a high level.

### Example 5 Clinical infusion of TIL and changes in TCR phenotype in vivo

In order to verify the clinical safety and effectiveness of the TIL obtained by the culture method of the present application, a clinical trial of TIL infusion therapy against solid tumors was conducted. This clinical trial has been approved by the Ethics Committee of Shanghai Tenth People's Hospital. For details, refer to http://www.chictr.org.cn/ under Registration No.: ChiCTR2100044705; or see www.clinicaltrials.gov under Registration No.: NCT04766320.

### Study protocol

1) Low-intensity lymphodepletion pretreatment: from Day 6 to Day 4 prior to TIL infusion, cyclophosphamide was administered to patients at a dose of 6 mg/kg/day for three consecutive days, and from Day 3 to Day 1 prior to infusion, fludarabine was administrated to the patients at 5 mg/m²/day for three consecutive days.
2) On the day after fludarabine administration, 10¹⁰-10¹¹ autologous TILs were intravenously infused into the patients.
3) The statuses of patients after infusion were monitored and recorded. Peripheral blood was collected at various time points to detect the composition and changes of PBMCs. The effect was evaluated by imaging 1-3 months after infusion.

### Preparation of TILs for infusion

1) TIL seed cells derived from surgically resected tumor tissues of patients were prepared according to the method described in the aforementioned Example 2;
2) Preparation of expansion medium: according to the formula and method described in Example 5 of the Chinese Application No. CN110099998A, the AIM-V medium was supplemented with IL-2 on the day of use, and the final concentration was reduced from 3000 IU/mL to 1000 IU/mL; and according to the concentration described in Example 6 of CN110099998A, the AIM-V medium containing 1000 IU/mL IL-2 was supplemented with a CD3 antibody (OKT3) to a final concentration of 30 ng/mL, which was used as an expansion medium for the expansion of TILs;
3) Preparation of infusion product (IP): 1×10⁸ seed cells cultured from the aforementioned surgically resected tumor tissues were used, and expanded and cultured for 12-14 days at 37°C in the presence of 5% CO₂ in a G-REX100 culture tank by using 1000 mL of expansion medium prepared in step 2); and the cells were harvested, centrifuged, washed with D-PBS, resuspended in normal saline, and released after quality control tests (including the percentage of CD3⁺, the percentages of CD4⁺ and CD8⁺, an IFN-γ secretion level, sterility, endotoxin, osmotic pressure, etc.) demonstrated that all indicators were in line with the requirements of the Pharmacopoeia of the People's Republic of China or the corresponding general standards of immune cell therapy products that were already on the market in China and abroad, such that the infusion product was obtained.

The aforementioned entire preparation process involving the TIL infusion product was carried out in a GMP-level production workshop in accordance with the requirements of relevant laws and regulations.

Infusion for subject T015 (with recurrent cervical cancer): The T015 TIL seed cells obtained in Example 2 were used for expansion and culture according to the above method, to finally obtain 4×10¹⁰ cells for the infusion product; and the cells were resuspended in 200 mL of normal saline, and intravenously and autologously infused to patients pretreated by low-intensity lymphodepletion pretreatment, without medicinal IL-2 injection after infusion. Before infusion, on Day 19 (D19), Day 30 (D30) and Day 47 (D47) after infusion, respectively, the peripheral blood of the patients were collected and detected in terms of PBMC composition and changes; and meanwhile, the PBMCs at different time points were isolated, and together with the IP, were subjected to high-throughput sequencing analysis with respect to the TCR repertoire.

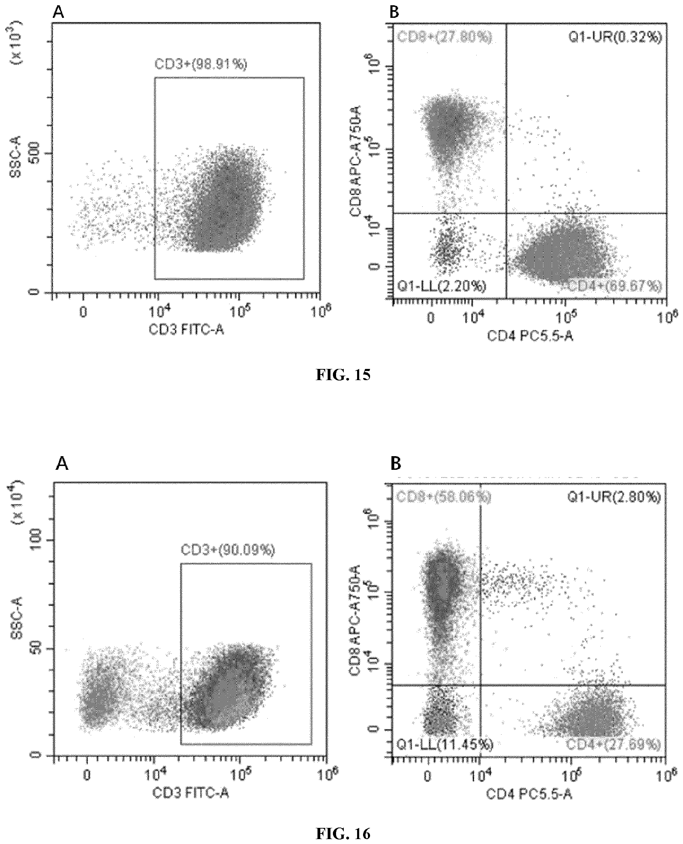

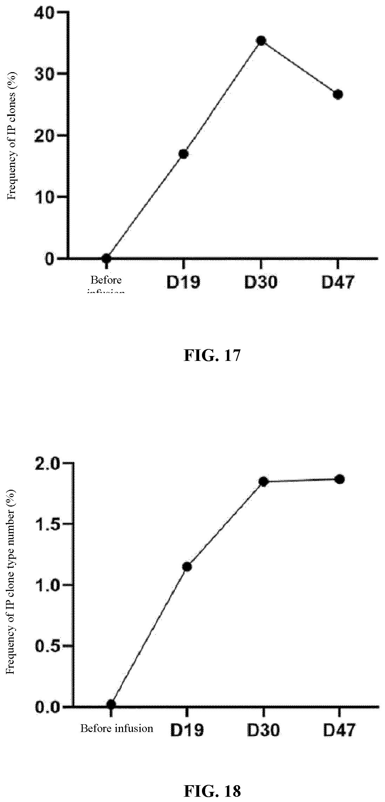

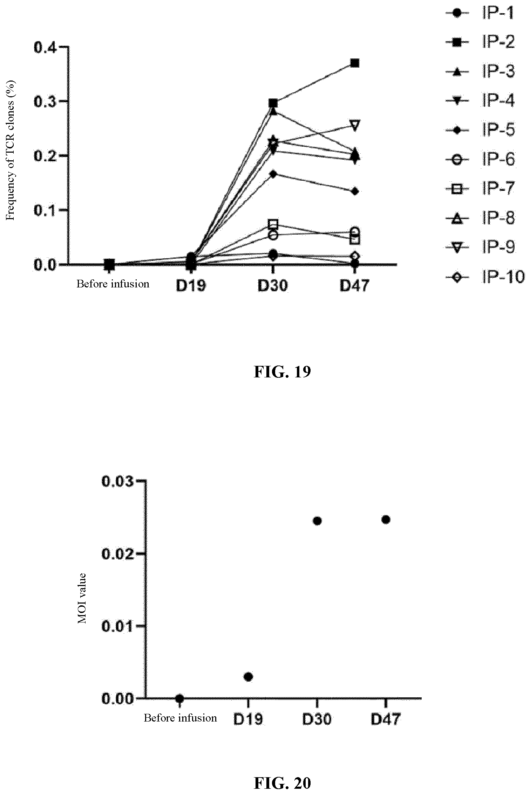

The results were shown in FIG. 16 to FIG. 20. FIG. 16 showed that the percentage of CD3⁺ cells in the infusion product exceeded 90%, and the ratio of CD8⁺ cells to CD4⁺ cells in the CD3⁺ cells exceeded 2: 1. FIG. 17 showed that the frequency of TCR clones (i.e., IP clones) of the infusion product TIL in the patient's peripheral blood began to rise after infusion, reaching a peak on D30, and decreasing on D47 compared with that on D30. FIG. 18 showed that the frequency of IP clonotypes in the patient's peripheral blood began to rise after infusion, reaching a peak on D30, and did not exhibit significant decrease on D47 compared with that on D30. FIG. 19 showed that the frequency of most of the top 10 TCR clones (named IP-1-IP-10, respectively) in the infusion product had a significant increase after infusion, most reaching a peak on D30, and the frequencies of TCR clones ranking second and ninth continued to rise after D30. FIG. 20 showed the Morisita index (MOI) values between the TCR repertoire of IP and the TCR repertoires of peripheral blood T cells before infusion and on D19, D30 and D47, respectively. As time after infusion elapsed, the MOI between the TCR repertoire of the peripheral blood T cells and the TCR repertoire of the infusion product gradually increased, reaching a peak on D30, and did not show significant decrease on D47.

The above results indicated that the TIL seed cells cultured using the TIL seed medium of the present application could reach the magnitude order of 10¹⁰-10¹¹ cells after expansion and culture, and after autologous infusion into tumor patients pretreated with low-intensity lymphodepletion, these TIL seed cells could proliferate significantly in vivo for a long time, without the administration of IL-2.

### Example 6 Clinical infusion of TIL and in vivo cell proliferation

### Study protocol

1) Low-intensity lymphodepletion pretreatment: from Day 3 to Day 1 prior to TIL infusion, cyclophosphamide was administered to patients at a dose of 6 mg/kg/day for three consecutive days, and fludarabine was not administrated.
2) On the day after cyclophosphamide administration, 10¹⁰-10¹¹ autologous TILs were intravenously infused into the patients.
3) The statuses of patients after infusion were monitored and recorded. Peripheral blood was collected at various time points to detect the composition and changes of PBMCs. The effect was evaluated by imaging 1-3 months after infusion.

The TILs for infusion were prepared as in Example 5.

Infusion for subject T016 (a patient with cervical cancer): The T016 TIL seed cells obtained in Example 2 were selected for expansion and culture according to the method in Example 5, to finally obtain 5.2×10¹⁰ cells for the infusion product; and the cells were resuspended in 250 mL of normal saline, and intravenously and autologously infused into the patient pretreated by low-intensity lymphodepletion, without medicinal IL-2 injection after infusion. On Day 4, Day 3 (cyclophosphamide administered), D2 (cyclophosphamide administered) and D1 (cyclophosphamide administered) before infusion and on Day 1, Day 6, Day 8, Day 13 and Day 17 after infusion, respectively, the peripheral blood was collected from the test subject, and number of leukocytes, neutrophils and lymphocytes were quantitated.

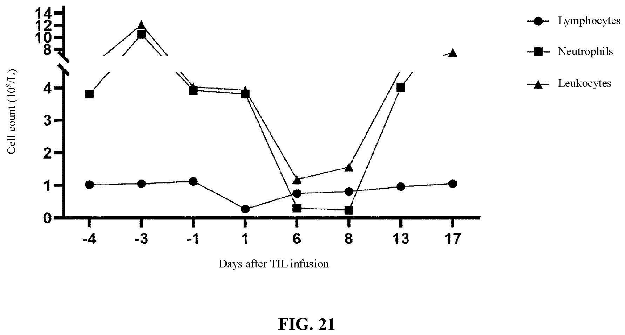

Results are shown in FIG. 21. FIG. 21 showed that around 10 days (Day 6 to Day 8 after infusion) after pretreatment with cyclophosphamide, the number of leukocytes and neutrophils in the peripheral blood of the test subject decreased to the lowest level, which was consistent with publication (M E Gershwin, E J Goetzl, A D Steinberg Cyclophosphamide: use in practice Ann Intern Med. 1974 Apr;80(4):531-40). The level of lymphocytes in the peripheral blood decreased to the lowest level on Day 1 after infusion since the pretreatment with cyclophosphamide, but began to rise continuously from Day 1 after infusion; and even though cyclophosphamide was still taking effect, and the overall level of neutrophils and white blood cells was at the bottom, there was still a significant increase in the number of lymphocytes; and by Day 13 after infusion, the lymphocytes had basically returned to the level before the pretreatment with cyclophosphamide. The above results indicated that the TIL seed cells cultured using the TIL seed medium of the present application could reach the order of magnitude of 10¹⁰-10¹¹ cells after expansion, and showed robust and continuous proliferation of lymphocytes in vivo after autologous infusion into the patient.

The embodiments of the present application are described in detail above. However, the present application is not limited to the concrete details in the aforementioned embodiments. Within the scope of the technical concept of the present application, a variety of simple variations can be made to the technical solution of the present application, and these simple variations should fall within the protection scope of the present application. In addition, it should be noted that the various specific technical features described in the aforementioned specific embodiments can be combined in any suitable manner without contradiction. In order to avoid unnecessary repetition, the present application will not explain various possible combination methods separately. Furthermore, the various different embodiments of the present application can also be combined arbitrarily, as long as they do not violate the idea of the present application, and they should also be deemed as the content disclosed in the present application.

## Claims

1. A seed cell medium for tumor-infiltrating lymphocytes, comprising the following components: a cell culture ingredient, a cytokine, and an immune checkpoint antibody or an antigen-binding fragment thereof, wherein said cytokine comprises IL-2, said immune checkpoint comprises: PD-1, LAG-3, TIGIT and/or CTLA-4, said cell culture ingredient is a serum-containing medium or a serum-free medium.

2. The seed cell medium according to claim 1, wherein said IL-2 has a concentration of about 3000 IU/mL or less.

3. The seed cell medium according to any one of claims 1-2, wherein said IL-2 has a concentration of about 2000 IU/mL to about 3000 IU/mL.

4. The seed cell medium according to any one of claims 1-3, wherein said cytokine comprises IL-7.

5. The seed cell medium according to claim 4, wherein said IL-7 has a concentration of about 200 U/mL to about 1000 U/mL.

6. The seed cell medium according to any one of claims 1-5, wherein said cytokine comprises IL-15.

7. The seed cell medium according to claim 6, wherein said IL-15 has a concentration of about 200 U/mL to about 500 U/mL.

8. The seed cell medium according to any one of claims 1-7, wherein said cytokine comprises a tumor necrosis factor TNF.

9. The seed cell medium according to any one of claims 1-8, wherein said cytokine comprises TNFα.

10. The seed cell medium according to claim 9, wherein said TNFα has a concentration of about 10 pg/mL to about 100 pg/mL.

11. The seed cell medium according to any one of claims 1-10, wherein said cytokine comprises a colony stimulating factor.

12. The seed cell medium according to any one of claims 1-11, wherein said cytokine comprises GM-CSF, G-CSF and/or M-CSF.

13. The seed cell medium according to claim 12, wherein said GM-CSF has a concentration of about 200 U/mL to about 5000 U/mL.

14. The seed cell medium according to any one of claims 12-13, wherein said G-CSF has a concentration of about 300 U/mL to about 1000 U/mL.

15. The seed cell medium according to any one of claims 12-14, wherein said M-CSF has a concentration of about 300 U/mL to about 800 U/mL.

16. The seed cell medium according to any one of claims 1-15, wherein said cytokine comprises an interferon.

17. The seed cell medium according to any one of claims 1-16, wherein said cytokine comprises IFN-γ, IFN-α and/or IFN-β.

18. The seed cell medium according to claim 17, wherein said IFN-γ has a concentration of about 10 U/mL to about 1000 U/mL.

19. The seed cell medium according to any one of claims 17-18, wherein said IFN-α has a concentration of about 500 U/mL to about 1000 U/mL.

20. The seed cell medium according to any one of claims 17-19, wherein said IFN-β has a concentration of about 200 U/mL to about 500 U/mL.

21. The seed cell medium according to any one of claims 1-20, wherein said cytokine further comprises: IL-4, IL-1α, IL-1β, IL-6, IL-9, IL-18, IL-12, IL-21 and/or IL-10.

22. The seed cell medium according to claim 21, wherein said IL-4 has a concentration of about 200 U/mL to about 500 U/mL.

23. The seed cell medium according to any one of claims 21-22, wherein said IL-1α has a concentration of about 200 U/mL to about 500 U/mL.

24. The seed cell medium according to any one of claims 21-23, wherein said IL-1β has a concentration of about 200 U/mL to about 500 U/mL.

25. The seed cell medium according to any one of claims 21-24, wherein said IL-6 has a concentration of about 200 U/mL to about 500 U/mL.

26. The seed cell medium according to any one of claims 21-25, wherein said IL-9 has a concentration of about 200 U/mL to about 500 U/mL.

27. The seed cell medium according to any one of claims 21-26, wherein said IL-18 has a concentration of about 200 U/mL to about 500 U/mL.

28. The seed cell medium according to any one of claims 21-27, wherein said IL-12 has a concentration of about 200 U/mL to about 500 U/mL.

29. The seed cell medium according to any one of claims 21-28, wherein said IL-21 has a concentration of about 200 U/mL to about 500 U/mL.

30. The seed cell medium according to any one of claims 21-29, wherein said IL-10 has a concentration of about 200 U/mL to about 500 U/mL.

31. The seed cell medium according to any one of claims 1-30, wherein said immune checkpoint antibody or the antigen-binding fragment thereof is a human PD-1 antibody or an antigen-binding fragment thereof.

32. The seed cell medium according to any one of claims 1-31, wherein said immune checkpoint antibody or the antigen-binding fragment thereof is a PD-1 antibody or an antigen-binding fragment thereof, and said PD-1 antibody or the antigen-binding fragment thereof has a concentration of about 1 µg/mL to about 100 µg/mL.

33. The seed cell medium according to any one of claims 1-32, wherein said immune checkpoint antibody or the antigen-binding fragment thereof is a human LAG-3 antibody or an antigen-binding fragment thereof.

34. The seed cell medium according to any one of claims 1-33, wherein said immune checkpoint antibody or the antigen-binding fragment thereof is an LAG-3 antibody or an antigen-binding fragment thereof, and said LAG-3 antibody or the antigen-binding fragment thereof has a concentration of about 3 µg/mL to about 10 µg/mL.

35. The seed cell medium according to any one of claims 1-34, wherein said TIGIT antibody or the antigen-binding fragment thereof is a human TIGIT antibody or an antigen-binding fragment thereof.

36. The seed cell medium according to any one of claims 1-35, wherein said immune checkpoint antibody or the antigen-binding fragment thereof is a TIGIT antibody or an antigen-binding fragment thereof, and said TIGIT antibody or the antigen-binding fragment thereof has a concentration of about 1 µg/mL to about 25 µg/mL.

37. The seed cell medium according to any one of claims 1-36, wherein said immune checkpoint antibody or the antigen-binding fragment thereof is a human CTLA-4 antibody or an antigen-binding fragment thereof.

38. The seed cell medium according to any one of claims 1-37, wherein said immune checkpoint antibody or the antigen-binding fragment thereof is a CTLA-4 antibody or an antigen-binding fragment thereof, and said CTLA-4 antibody or the antigen-binding fragment thereof has a concentration of about 1 µg/mL to about 100 µg/mL.

39. The seed cell medium according to any one of claims 1-38, further comprising a costimulatory receptor antibody or an antigen-binding fragment thereof, wherein said costimulatory receptor antibody or the antigen-binding fragment thereof comprises: a CD40 antibody or an antigen-binding fragment, an OX-40 antibody or an antigen-binding fragment thereof, a CD137 antibody or an antigen-binding fragment thereof, and/or a CD28 antibody thereof or an antigen-binding fragment thereof.

40. The seed cell medium according to claim 39, wherein said CD137 antibody or the antigen-binding fragment thereof is a human CD137 antibody or an antigen-binding fragment thereof.

41. The seed cell medium according to any one of claims 39-40, wherein said CD137 antibody or the antigen-binding fragment thereof has a concentration of about 1 µg/mL to about 100 µg/mL.

42. The seed cell medium according to any one of claims 39-41, wherein said CD28 antibody or the antigen-binding fragment thereof is a human CD28 antibody or an antigen-binding fragment thereof.

43. The seed cell medium according to any one of claims 39-42, wherein said CD28 antibody or the antigen-binding fragment thereof has a concentration of about 1 µg/mL to about 10 µg/mL.

44. The seed cell medium according to any one of claims 39-43, wherein said CD40 antibody or the antigen-binding fragment thereof is a human CD40 antibody or an antigen-binding fragment thereof.

45. The seed cell medium according to any one of claims 39-44, wherein said CD40 antibody or the antigen-binding fragment thereof has a concentration of about 5 µg/mL to about 10 µg/mL.

46. The seed cell medium according to any one of claims 39-45, wherein said OX-40 antibody or the antigen-binding fragment thereof is a human OX-40 antibody or an antigen-binding fragment thereof.

47. The seed cell medium according to any one of claims 39-46, wherein said OX-40 antibody or the antigen-binding fragment thereof has a concentration of about 3 µg/mL to about 10 µg/mL.

48. The seed cell medium according to any one of claims 1-47, wherein said serum-containing medium comprises a serum.

49. The seed cell medium according to claim 48, wherein said serum comprises a human AB serum.

50. The seed cell medium according to any one of claims 48-49, wherein said serum has a concentration of about 1% to about 10% (v/v).

51. The seed cell medium according to any one of claims 1-50, wherein said serum-containing medium comprises an AIM-V medium.

52. The seed cell medium according to any one of claims 1-51, wherein said serum-free medium comprises an X-VIVO medium.

53. The seed cell medium according to any one of claims 1-52, wherein said cell culture ingredient comprises antibiotics.

54. The seed cell medium according to any one of claims 1-53, wherein said cell culture ingredient comprises a penicillin-streptomycin PS mixed solution.

55. The seed cell medium according to claim 54, wherein said penicillin-streptomycin PS mixed solution has a concentration of about 1 U/mL to about 200 U/mL.

56. The seed cell medium according to any one of claims 1-55, further comprising an M2 macrophage inhibitor, said M2 macrophage inhibitor comprising RRx001 and/or CNI-1493.

57. The seed cell medium according to claim 56, wherein said M2 macrophage inhibitor has a concentration of about 0.1 µM to about 100 µM.

58. The seed cell medium according to any one of claims 1-57, further comprising a regulatory T cell (Treg) inhibitor, said regulatory T cell inhibitor comprising CAL-101, dasatinib, imatinib and/or panobinostat.

59. The seed cell medium according to claim 58, wherein said regulatory T cell inhibitor has a concentration of about 0.1 µM to about 100 µM.

60. The seed cell medium according to any one of claims 1-59, further comprising a myeloid-derived suppressor cell inhibitor, said myeloid-derived suppressor cell inhibitor comprising AG490, decitabine, sunitinib and/or BBI608.

61. The seed cell medium according to claim 60, wherein said myeloid-derived suppressor cell inhibitor has a concentration of about 0.1 µg/mL to about 100 µg/mL.

62. The seed cell medium according to any one of claims 1-61, further comprising a T cell activator, said T cell activator comprising LYC-55716, GNE-1858 and/or methylene blue.

63. The seed cell medium according to claim 62, wherein said T cell activator has a concentration of about 1 µM to about 10 µM.

64. The seed cell medium according to any one of claims 1-63, further comprising a T cell differentiation inhibitor, said T cell differentiation inhibitor comprising TWS119.

65. The seed cell medium according to claim 64, wherein said T cell differentiation inhibitor has a concentration of about 1 µM to about 10 µM.

66. A seed cell of a tumor-infiltrating lymphocyte obtained by culturing using said seed cell medium according to any one of claims 1-65, or a cell population thereof.

67. A pharmaceutical composition, comprising said seed cell of the tumor-infiltrating lymphocyte or the cell population thereof according to claim 66, and a pharmaceutically acceptable carrier.

68. Use of said seed cell of the tumor-infiltrating lymphocyte or the cell population thereof according to claim 66 and said pharmaceutical composition according to claim 67 in the preparation of a medicament for treating a cancer.

69. The use according to claim 68, wherein said cancer is selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated pelvic adenocarcinoma, and bile duct cancer.

70. Use of said seed cell medium according to any one of claims 1-65 in the expansion of tumor-infiltrating lymphocytes.

71. A method for culturing a seed cell for tumor-infiltrating lymphocytes, comprising the steps of: culturing isolated tumor-infiltrating lymphocytes in the seed cell medium according to any one of claims 1-65.

72. A method for expanding tumor-infiltrating lymphocytes, comprises the steps of: culturing isolated tumor-infiltrating lymphocytes in said seed cell medium according to any one of claims 1-65 to obtain seed cells of said tumor-infiltrating lymphocytes.

73. The method according to any one of claims 71-72, wherein said isolated tumor-infiltrating lymphocytes are derived from a sample selected from the group consisting of: an ascite from a test subject in need, a primary tumor sample from surgical resection, a metastasis sample from synchronous and metachronous surgical resection, a needle biopsy sample, and a body fluid.

74. The method according to claim 73, wherein said body fluid comprises blood, a tissue fluid, a lymph fluid and/or a body cavity effusion.

75. The method according to any one of claims 71-74, wherein said isolated tumor-infiltrating lymphocytes are derived from a tumor selected from the group consisting of: melanoma, glioma, gastric cancer, lung cancer, gastrointestinal stromal tumor, intestinal cancer, liver cancer, cervical cancer, ovarian cancer, breast cancer, endometrial stromal sarcoma, poorly differentiated pelvic adenocarcinoma, and bile duct cancer.

76. The method according to any one of claims 71-75, wherein said isolated tumor-infiltrating lymphocytes are derived from tissue fragments obtained by cutting a tumor tissue resection, and said tissue fragments obtained by cutting a tumor tissue resection has a diameter of about 1 mm to about 10 mm.

77. The method according to any one of claims 71-76, wherein a time for said culturing is about 3-20 days.

78. The method according to any one of claims 71-77, wherein a temperature for said culturing is about 30-42°C.
